# EUROPÄISCHE PATENTSCHRIFT

(11) **EP 4 179 060 B1**
(45) Veröffentlichungstag und Bekanntmachung des Hinweises auf die Patenterteilung: **08.07.2026**
(21) Anmeldenummer: 21749116.6
(22) Anmeldetag: 13.07.2021
(51) Int. Cl.: C12M 1/36, B61D 35/00, C02F 3/00, C02F 3/12, C02F 103/00, C12M 1/00

(54) **BIOREAKTORREINIGUNGSANLAGE FÜR BIOREAKTOREN IN SCHIENENFAHRZEUGEN**
BIOREACTOR CLEANING INSTALLATION FOR BIOREACTORS IN RAIL VEHICLES
INSTALLATION DE NETTOYAGE DE BIORÉACTEUR POUR BIORÉACTEURS DANS DES VÉHICULES SUR RAILS

(30) Priorität: 13.07.2020 DE 202020104037 U; 28.07.2020 DE 102020119924
(43) Veröffentlichungstag der Anmeldung: 17.05.2023
(73) Patentinhaber: Vogelsang GmbH & Co. KG, 49632 Essen (DE)
(72) Erfinder: HEINRICHS, Martin, 49632 Essen (DE)
(74) Vertreter: Eisenführ Speiser
(86) Internationale Anmeldenummer: PCT/EP2021/069411
(87) Internationale Veröffentlichungsnummer: WO 2022/013194

(56) Entgegenhaltungen:
- EP-A1- 2 098 280
- EP-A1- 2 364 893
- EP-A1- 2 789 683
- CN-A- 110 790 452
- DD-A1- 140 488
- DE-A1- 102016 115 393
- DE-A1- 19 524 960
- DE-U1- 202017 101 065
- SÜDOSTBAHN ET AL: "Das stille Örtchen wird aufgerüstet", 26 June 2017 (2017-06-26), St. Gallen, pages 1 - 2, XP055849569, Retrieved from the Internet <URL:https://www.sob.ch/fileadmin/images/medienmitteilungen/2017/MM_Bioreaktor-26-6-2017.pdf> [retrieved on 20211008]

## Beschreibung

Die Erfindung betrifft eine Bioreaktorreinigungsanlage zum Reinigen eines Bioreaktors, vorzugsweise eines Bioreaktors in einem Schienenfahrzeug, mit einem ersten Absauganschluss zum Anschließen an den Bioreaktor, einem zweiten Absauganschluss zum Anschließen an den Bioreaktor, über den eine Flüssigkeit aus einem Filterkorb des Bioreaktors absaugbar ist, einem Spülanschluss zum Zuführen einer Flüssigkeit zu einer Reinigungsdüse des Bioreaktors, einem Säuretank zum Aufnehmen einer wässrigen Säurelösung, einem Sammeltank zum Aufnehmen von aus dem Bioreaktor abgesaugter Flüssigkeit, einem Frischwasseranschluss zum Versorgen der Bioreaktorreinigungsanlage mit Frischwasser und einer Pumpe mit einem ersten Pumpanschluss und einem zweiten Pumpanschluss. Bei stationären Anlagen kann der Sammeltank auch entfallen und die aus dem Bioreaktor abgesaugte Flüssigkeit direkt einem Kanalabfluss zugeführt werden. Die Erfindung betrifft ferner ein Verfahren zum Betreiben einer solchen Bioreaktorreinigungsanlage, insbesondere um einen Bioreaktor zu warten und/oder zu reinigen, sowie ein Computerprogramm zum Steuern der Bioreaktorreinigungsanlage.

Herkömmliche Bioreaktoren verfügen über einen Feststofftank mit einem Filterkorb, in den Abwasser mit festen und flüssigen Bestandteilen eingeleitet wird. Der Filterkorb trennt die festen von den flüssigen Bestandteilen. Dafür weist der Filterkorb an den begrenzenden Wänden, wie Boden- und Seitenwänden, Filterelemente auf, durch welche flüssige Elemente abfließen können und durch welche feste Elemente aufgefangen werden. Die festen Elemente sammeln sich am Boden innerhalb des Filterkorbs separiert von den flüssigen Elementen und bilden einen Filterkuchen. Die flüssigen Elemente fließen durch die Filterelemente hindurch in den Feststofftank und von dort in einen Flüssigtank, der in Fluidverbindung mit dem Feststofftank steht.

EP 2 789 683 offenbart eine Bioreaktorreinigungsanlage zum Reinigen eines Bioreaktors mit einigen Anschlüssen, beispielsweise zum Ablassen, Absaugen, Einfüllen von Reinigungslösung und eventuell Säure- und Baselösungen aus einem oder mehrerer Tanks.

Es ist bekannt, dass sich die festen Elemente in dem Filterkorb als Filterkuchen absetzen. Zuerst bildet sich ein Filterkuchen beginnend an einer Bodenseite des Filterkorbs und anschließend an den Seiten des Filterkorbs. Dadurch wird das Wasser durch den Filterkuchen gehemmt, in den Feststofftank abzufließen. Ein Filterkuchen mit gewisser Durchlässigkeit führt zu einem effizienten Filtervorgang. Jedoch kann ein zunehmend dicker und undurchlässiger werdender Filterkuchen dazu führen, dass der Filterkorb verstopft. Dies führt zu einem ineffizienten Filterverfahren, da die Flüssigkeit kaum mehr durch den Filter gelangt. Es ist daher notwendig, den Filterkorb in regelmäßigen Abständen von Feststoffen zu reinigen, um einen ausreichenden Abfluss des Wassers in den Feststofftank zu gewährleisten.

Es ist bekannt, den Filterkuchen zu entfernen, um diesem Verstopfen zu begegnen. Oftmals wird hierbei der Filterkuchen entfernt, sobald erste Effekte einer Verstopfung auftreten. Dies hat allerdings den Nachteil, dass bereits eine ineffiziente Filterung stattgefunden hat. Es ist auch bekannt, von Zeit zu Zeit die Menge des Filterkuchens zu überprüfen, um daran festzustellen, ob eine Entfemung notwendig ist. Dies hat allerdings den Nachteil, dass die Überprüfung zufällig erfolgt, und der richtige Zeitpunkt, also weder zu früh noch zu spät, für eine Entfernung des Filterkuchens so nicht zuverlässig getroffen werden kann. Zudem ist eine Beurteilung, ob der Filterkuchen bereits so undurchlässig ist, dass er entfernt werden muss, nicht zuverlässig möglich.

Ein Problem bei solchen Reinigungsvorgängen liegt jedoch darin, dass Bioreaktoranlagen in der Regel als geschlossenes System aufgebaut sind und daher eine Ermittlung des Verschmutzungsgrades und der Ursache für eine unzureichende Filterung nur sehr aufwendig möglich ist. Gerade schon bestehende Bioreaktoren verfügen häufig nicht über Schnittstellen, mittels derer für die Bestimmung einer Fehlerursache oder eines Verschmutzungsgrades notwendige Informationen oder auch nur dafür hilfreiche Daten ausgelesen werden können. Dies ist insbesondere dann zusätzlich erschwert, wenn ein solcher Bioreaktor an Bord eines Fahrzeugs, wie beispielsweise eines gleisgebundenen Waggons, eingebaut ist, um das dort anfallende Schmutzwasser zu reinigen. In solchen Anwendungsfällen ist eine Wartung und Sicherstellung der Funktion des Bioreaktors häufig dezentral und ohne dessen Ausbau gewünscht, zugleich aber aufgrund der notwendigen Kompaktheit der Zugang zum Bioreaktor und zu Daten, die dessen Zustand beschreiben, nicht oder nur sehr aufwendig möglich.

Zudem bestehen Probleme beim Durchführen von Reinigungen. Die Handhabung zwischen verschiedenen Bedienern schwankt und so kann das Reinigungsergebnis von dem Können des jeweiligen Bedieners abhängen.

Eine Aufgabe der vorliegenden Erfindung ist es daher, eine Bioreaktorreinigungsanlage der eingangs genannten Art anzugeben, welche einfach aufgebaut ist, wenig fehleranfällig, vielseitig einsetzbar und möglichst eine gleichmäßige und stetig gute Reinigung eines Bioreaktors erlaubt.

Die Erfindung löst diese Aufgabe bei einer Bioreaktorreinigungsanlage der eingangs genannten Art mit den Merkmalen des Anspruchs 1, nämlich insbesondere dadurch, dass einerseits eine Messeinheit zum Messen von Flüssigkeit vorgesehen ist, und wobei mittels der Pumpe Flüssigkeit von dem ersten Absauganschluss wahlweise in die Messeinheit, in den Sammeltank oder den Säuretank pumpbar ist, eine wässrigen Säurelösung aus dem Säuretank zum zweiten Absauganschluss und/oder zum Spülanschluss pumpbar ist, Frischwasser von dem Frischwasseranschluss wahlweise zu dem Spülanschluss oder der Messeinheit pumpbar ist, und Flüssigkeit aus der Messeinheit wahlweise in den Sammeltank oder zum zweiten Absauganschluss pumpbar ist. Bei stationären Anlagen kann der Sammeltank auch entfallen und die aus dem Bioreaktor abgesaugte Flüssigkeit direkt einem Kanalabfluss zugeführt werden.

Der Erfindung liegt die Erkenntnis zugrunde, dass einerseits durch geschickte Verschaltung von den einzelnen Elementen und funktionalen Einheiten der Bioreaktorreinigungsanlage die Funktionalität der Bioreaktorreinigungsanlage insgesamt verbessert und erweitert werden kann und andererseits die Messeinheit zum Messen der Flüssigkeit Funktionen, wie insbesondere das Testen einer Durchlässigkeit des Bioreaktors, das Reinigen des Bioreaktors mit einer unter Druck stehenden Flüssigkeit, wie beispielsweise Frischwasser, sowie das Reinigen des Bioreaktors mit einer Chemikalie, wie insbesondere einer wässrigen Säurelösung, umsetzbar sind. Die Verschaltung der einzelnen Elemente erlaubt einen automatisierten Betrieb bzw. weitgehend automatisierten Betrieb von der Bereitstellung einer wässrigen Säurelösung bis hin zum Testen der Durchlässigkeit des Bioreaktors.

Der Begriff Flüssigkeit wird in der vorliegenden Offenbarung als Oberbegriff für Stoffe wie Frischwasser, wässrige Säurelösungen, Säure, Brauchwasser aber viskosere Medien, insbesondere Schlamm, und dergleichen verstanden. Der Begriff wässrige Säurelösung bezeichnet in der vorliegenden Offenbarung eine Flüssigkeit, die ein Gemisch aus einer Säure und Wasser ist.

Die Pumpe ist vorzugsweise eine Drehkolbenpumpe, die dazu ausgebildet ist, sowohl vom ersten Pumpanschluss zum zweiten Pumpanschluss zu pumpen, also auch umgekehrt vom zweiten Pumpanschluss zum ersten Pumpanschluss. Auch andere Pumpenarten sind denkbar bevorzugt, wie beispielsweise peristaltische Pumpen oder dergleichen. Drehkolbenpumpen haben den Vorteil, dass auch mit Feststoffen beladende Fluide ohne Weiteres gepumpt werden können, bei ausreichendem Dichtheitsgrad und hoher Verschleißsicherheit.

Der Sammeltank ist vorzugsweise dazu vorgesehen, sämtliche Flüssigkeiten aufnehmen zu können. Dies betrifft einerseits aus dem Bioreaktor abgesaugte Flüssigkeit, das heißt Flüssigkeiten, die bereits vor Beginn des Reinigungsprozesses im Bioreaktor vorhanden sind, wie auch Flüssigkeiten, die aufgrund der Reinigung des Bioreaktors in eingebracht wurden. Wird beispielsweise eine mechanische Reinigung des Bioreaktors mittels einer in dem Bioreaktor angeordneten Düse und unter Druck stehendem Frischwasser durchgeführt, kann die anschließend im Bioreaktor vorhandene Flüssigkeit abgesaugt und im Sammeltank gespeichert werden. Der Sammeltank dient so als ein Zwischenspeicher und die darin vorhandene Flüssigkeit kann nach Beendigung der Reinigung entsorgt werden. In dem Säuretank ist vorzugsweise wässrige Säurelösung gespeichert. Diese wird dazu verwendet, eine chemische Reinigung des Bioreaktors, wie insbesondere eine Entfernung von Kalkablagerung, durchzuführen. Nach erfolgter chemischer Reinigung wird die wässrige Säurelösung wieder aus dem Bioreaktor abgesaugt und kann dann, wenn sie noch eine ausreichende Qualität hat, um in einem weiteren Reinigungsprozess verwendet zu werden, wiederum im Säuretank gespeichert werden. Soll die wässrige Säurelösung allerdings entsorgt werden, kann sie zu Neutralisierungszwecken zunächst im Säuretank neutralisiert werden, oder direkt in den Sammeltank gegeben werden, um anschließend an einem geeigneten Ort unter geeigneten Bedingungen entsorgt zu werden.

Die einzelnen Anschlüsse der Bioreaktorreinigungsanlage sind vorzugsweise mit entsprechenden Kupplungen ausgestattet, wie beispielsweise Camlock-Kupplungen oder dergleichen. Bioreaktoren, die in Zügen vorgesehen sind, haben Standardanschlüsse, an denen die Bioreaktorreinigungsanlage gemäß der hierin beschriebenen Erfindung anschließbar ist.

In einer ersten bevorzugten Ausführungsform weist die Bioreaktorreinigungsanlage eine elektronische Steuereinheit auf, wenigstens zum Steuern der Pumpe, wobei die elektronische Steuereinheit einen Speicher und einen Prozessor aufweist und dazu ausgebildet ist, wenigstens einen ersten Parameter von wenigstens einem Sensor der Bioreaktorreinigungsanlage und/oder wenigstens einen zweiten Parameter von einen Benutzer über die Mensch-Maschine-Schnittstelle zu empfangen, und wobei die elektronische Steuereinheit die Pumpe basierend auf den ersten und zweiten Parametern ansteuert. Darüber hinaus kann die elektronische Steuereinheit dazu eingerichtet sein, weitere Parameter zu erfassen, wie insbesondere Parameter des Bioreaktors, die vorzugsweise mittels der Bioreaktorreinigungsanlage aus dem Bioreaktor ausgelesen werden. Zu diesem Zweck kann eine elektronische Schnittstelle einer Bioreaktorreinigungsanlage vorgesehen sein, über die die Bioreaktorreinigungsanlage mit einer elektronischen Schnittstelle des Bioreaktors kommunizieren kann. Eine solche Schnittstelle ist vorzugsweise eine 7-polige Schnittstelle. Bioreaktoren weisen in der Regel fest verbaute Sensoren auf, wie beispielsweise Füllstandsensoren oder Sensoren, die einen Fehler des Bioreaktors ermitteln können. Neuere Bioreaktoren haben zudem eine Schnittstelle, die das Auslesen solcher Sensoren ermöglicht. Die Bioreaktorreinigungsanlage kann vorzugsweise derartige Sensoren auslesen und so zusätzlich Parameter des Bioreaktors verwenden, um die Pumpe und/oder Ventile anzusteuern. Die elektronische Steuereinheit ist vorzugsweise ferner dazu eingerichtet, ein oder mehrere Ventile der Bioreaktorreinigungsanlage anzusteuern, um so einen Strom von Flüssigkeit durch die Bioreaktorreinigungsanlage zu steuern. Sie ist vorzugswiese ferner mit einem oder mehreren Sensoren der Bioreaktorreinigungsanlage verbunden, um von diesen Signale zu empfangen. Auf diese Weise ist es möglich, eine weitergehende Automatisierung der Bioreaktorreinigungsanlage sowie eine verbesserte Reinigung des Bioreaktors zu erzielen.

Weiterhin ist bevorzugt, dass die Bioreaktorreinigungsanlage eine Säuredosiereinheit aufweist, die wenigstens einen Säurekanister-Anschluss zum Anschließen von einem oder mehreren Säurekanistern und wenigstens einen Basekanister-Anschluss zum Anschließen von einem oder mehreren Basekanistern aufweist und die mit dem Säuretank, dem Frischwasseranschluss und der Pumpe verbindbar ist. Der Begriff verbindbar bedeutet in diesem Zusammenhang, dass zwischen der Säuredosiereinheit, dem Säuretank, dem Frischwasseranschluss und der Pumpe noch weitere Elemente, insbesondere schaltbare Ventile, vorgesehen sein können. Die Säuredosiereinheit muss daher nicht zwingend unmittelbar mit den gennannten Elementen verbunden sein, sondern eine Verbindung über weitere Zwischenelemente ist ausreichend. Die Säuredosiereinheit dient dazu, wässrige Säurelösung herzustellen, indem sie Säure aus dem Säuretank und Frischwasser vom Frischwasseranschluss mischt, und diese Mischung in den Säuretank zuführt. Sie kann auch dazu eingesetzt werden, eine wässrige Säurelösung in dem Säuretank zu neutralisieren, indem sie Frischwasser vom Frischwasseranschluss Base aus dem oder den Basekanistern zumischt, und diese Mischung dann dem Säuretank zuführt. Dies kann erforderlich sein, um aus dem Bioreaktor abgesaugte wässrige Säurelösung zu neutralisieren, um diese dann sachgerecht entsorgen zu können.

Bevorzugt ist mittels der Pumpe Flüssigkeit vom Säuretank zur Dosiereinheit pumpbar. Auf diese Weise kann wässrige Säurelösung, oder andere Flüssigkeit, die im Säuretank vorhanden ist, zu der Säuredosiereinheit gepumpt werden, um dort mit Säure und/oder Base versetzt zu werden, um so den pH-Wert der in dem Säuretank vorhandenen Flüssigkeit einzustellen. Die Einstellung des pH-Werts der Flüssigkeit aus dem Säuretank findet dann nicht unmittelbar im Säuretank statt, sondern in der Dosiereinheit. Es kann auf diese Weise eine Durchlaufneutralisierung oder Durchlaufaufsäuerung der Flüssigkeit erreicht werden. Hierdurch ist es möglich, den pH-Wert genauer einzustellen, ohne einen pH-Gefälle innerhalb des Säuretanks zu provozieren.

Weiterhin ist bevorzugt, dass mittels der Pumpe Flüssigkeit vom ersten Absauganschluss zur Säuredosiereinheit pumpbar ist. Auf diese Weise kann Flüssigkeit, die aus dem Bioreaktor abgesaugt wurde, zunächst zur Säuredosiereinheit gepumpt werden, um von dort aus dann entweder in den Sammeltank oder den Säuretank gepumpt zu werden. Hierdurch ist es möglich, Flüssigkeit, die aus dem Bioreaktor abgesaugt wurde, zu neutralisieren oder aufzusäuern, und sie in Abhängigkeit davon entweder dem Säuretank oder dem Sammeltank zuzuführen. Alternativ kann auch eine Neutralisierung von saurer Flüssigkeit im Bioreaktor durchgeführt werden. Zu diesem Zweck wird die aus dem Bioreaktor abgesaugte Flüssigkeit wie zuvor beschrieben zur Säuredosiereinheit gepumpt, um dort mit Base angereichert zu werden. Sodann wird die Flüssigkeit aber nicht in den Säuretank oder Sammeltank geführt, sondern zurück in den Bioreaktor. Dieses Zyklieren der Flüssigkeit kann solange erfolgen, bis eine neutrale Flüssigkeit erreicht ist. Dabei wird jeweils so viel Base und/oder Säure zudosiert, wie nötig, um die Flüssigkeit zu neutralisieren.

In einer weiteren bevorzugten Ausführungsform ist vorgesehen, dass mittels der Pumpe Flüssigkeit von dem Säuretank zum Sammeltank pumpbar ist. Dies ist insbesondere dann bevorzugt, wenn in dem Säuretank eine bereits neutralisierte oder weitgehend neutralisierte Flüssigkeit vorliegt, die dann in den Sammeltank überführt wird, um von dort aus entsorgt werden zu können. Der Säuretank ist dann leer und bereit zum Aufnehmen einer neuen wässrigen Säurelösung. Typischerweise ist der Säuretank deutlich kleiner als der Sammeltank. Kommt es beispielsweise beim Reinigen eines Bioreaktors dazu, dass die wässrige Säurelösung eine nicht mehr ausreichende Qualität zum Reinigen des Bioreaktors hat, kann diese im Säuretank neutralisiert werden und anschließend wird der Säuretank in den Sammeltank entleert. In dem Säuretank kann dann eine neue wässrige Säurelösung erzeugt werden, um die Reinigung fortzusetzen. Ein Entleeren des Sammeltanks ist in diesem Fall zunächst noch nicht erforderlich und eine Unterbrechung des Reinigungsprozesses nicht nötig. Hierdurch kann die Reinigungszeit eines Bioreaktors verkürzt werden, Durchlaufzeiten verringert und insgesamt die Effizienz des Reinigungsprozesses erhöht werden.

Ferner weist die Bioreaktorreinigungsanlage vorzugsweise eine Hochdruckpumpe stromaufwärts des Spülanschlusses auf. Über den Spülanschluss wird vorzugsweise Frischwasser oder eine andere Flüssigkeit dem Bioreaktor unter Hochdruck bereitgestellt, um dort insbesondere eine mechanische Reinigung zu bewirken. Die Hochdruckpumpe kann für diesen Zweck gezielt an dem Spülanschluss eingesetzt werden. Auch die Hochdruckpumpe wird vorzugsweise von der elektronischen Steuereinheit gesteuert, wobei die elektronische Steuereinheit hierzu entsprechende Programmcode-Mittel aufweist.

In einer bevorzugten Weiterbildung ist vorgesehen, dass die Bioreaktorreinigungsanlage ein erstes Ventil aufweist, das den ersten Absauganschluss über eine erste Leitung mit einer zweiten Leitung verbindet. Vorzugsweise ist ein zweites Ventil vorgesehen, das die zweite Leitung mit dem ersten Pumpenanschluss verbindet. Die ersten und zweiten Ventile sind vorzugsweise mit der elektronischen Steuereinheit verbunden und können von dieser gesteuert werden. Über die ersten und zweiten Ventile ist der erste Absauganschluss mit dem ersten Pumpenanschluss verbunden oder verbindbar.

In einer weiteren bevorzugten Ausführungsform ist vorgesehen, dass die Bioreaktorreinigungsanlage ein drittes Ventil aufweist, das den zweiten Absauganschluss über eine dritte Leitung mit einer vierten Leitung verbindet. Der zweite Absauganschluss ist folglich mit der dritten Leitung verbunden, die dann mit dem dritten Ventil verbunden ist, das seinerseits die dritte Leitung mit der vierten Leitung verbindet. Vorzugsweise ist ein viertes Ventil vorgesehen, das die vierte Leitung mit dem zweiten Pumpenanschluss verbindet. Über die dritten und vierten Ventile ist also der zweite Absauganschluss mit dem zweiten Pumpenanschluss verbunden oder verbindbar. Auch die dritten und vierten Ventile sind vorzugsweise von der elektronischen Steuereinheit steuerbar.

Vorzugsweise ist ferner ein fünftes Ventil vorgesehen, das die zweite Leitung mit der vierten Leitung verbindet. Das fünfte Ventil bildet auf diese Weise einen Bypass für die Pumpe, über den entweder Flüssigkeit an der Pumpe vorbeigeleitet werden kann, andererseits auch eine Richtungsumkehr bezüglich des ersten Absauganschlusses und zweiten Absauganschluss vorgesehen sein kann. Das fünfte Ventil erlaubt so konkret, dass nicht nur der erste Absauganschluss mit dem ersten Pumpenanschluss verbunden oder verbindbar ist, sondern der erste Absauganschluss ist über das fünfte Ventil auch mit dem zweiten Pumpenanschluss verbindbar. Ebenso ist der zweite Absauganschluss nicht nur mit dem zweiten Pumpenanschluss verbindbar, sondern über das fünfte Ventil auch mit dem ersten Pumpenanschluss verbindbar.

In einer bevorzugten Weiterbildung ist der erste Pumpanschluss über ein erstes Sammeltankventil mit dem Sammeltank verbunden. Auch hier kann vorgesehen sein, dass zwischen dem Sammeltank und dem ersten Sammeltankventil bzw. zwischen dem ersten Pumpenanschluss und dem ersten Sammeltankventil weitere Elemente, wie beispielsweise Ventile oder dergleichen vorgesehen sein können. Durch Schalten des ersten Sammeltankventils ist also Flüssigkeit vom ersten Pumpenanschluss in den Sammeltank pumpbar, oder aus diesem heraus zum ersten Pumpenanschluss hin.

Ferner ist bevorzugt, dass der erste Pumpanschluss über ein erstes Säuretankventil mit dem Säuretank verbunden ist. Auch hier gilt, dass zwischen Pumpanschluss, Säuretankventil und Säuretank weitere Elemente vorhanden sein können, sodass auch eine mittelbare Verbindung möglich ist.

Weiter bevorzugt ist der zweite Pumpanschluss über ein zweites Sammeltankventil mit dem Sammeltank verbindbar oder verbunden. Vorzugsweise ist auch der zweite Pumpanschluss über ein zweites Säuretankventil mit dem Säuretank verbindbar oder verbunden. Auf diese Weise lässt sich auch ein Kreislauf vom Sammeltank in den Sammeltank hinein über die Pumpe sowie vom Säuretank in den Säuretank hinein über die Pumpe erzeugen, wodurch gewisse weitere Funktionen in Bezug auf die so gepumpte Flüssigkeit ausgeführt werden können, wie insbesondere das Messen von pH-Werten, das Messen von Flüssen, das Messen von Volumina und dergleichen.

Ferner weist die Bioreaktorreinigungsanlage vorzugsweise ein Rückführventil auf, das den zweiten Pumpanschluss mit der Säuredosiereinheit verbindet. Von dem zweiten Pumpanschluss kann demnach Flüssigkeit zur Säuredosiereinheit gepumpt werden oder umgekehrt.

Weiterhin ist bevorzugt, dass die Bioreaktorreinigungsanlage ein erstes Messventil aufweist, das den zweiten Pumpanschluss mit der Messeinheit verbindet. Über dieses erste Messventil kann von dem zweiten Pumpanschluss Flüssigkeit zur Messeinheit gepumpt werden, um dort innerhalb der Messeinheit das Volumen dieser Flüssigkeit zu messen. Hierdurch können einerseits bestimmte Volumina abgemessen werden, die anschließend dem Bioreaktor zugeführt werden, andererseits können auch Volumina, die aus dem Bioreaktor abgesaugt wurden, in der Messeinheit gemessen werden.

Ferner ist bevorzug ein zweites Messventil vorgesehen, das die Messeinheit mit einer ersten Messleitung verbindet. Das erste und das zweite Messventil münden vorzugsweise in unterschiedliche Anschlüsse an der Messeinheit, können aber auch an demselben Anschluss in die Messeinheit münden. Die erste Messleitung kann ebenso wie das erste Messventil auch zum zweiten Pumpanschluss führen, oder alternativ auch zum ersten Pumpanschluss. Vorzugsweise führt die erste Messleitung aber nicht unmittelbar zum ersten Pumpanschluss, sondern über wenigstens ein oder zwei Zwischeneinheiten, wie weitere Ventile. Beispielsweise kann das erste Messventil dazu verwendet werden, Flüssigkeit in die Messeinheit einzubringen, und das zweite Messventil dazu, Flüssigkeit aus der Messeinheit herauszubefördern.

Hierbei ist bevorzugt, dass die Bioreaktorreinigungsanlage ein sechstes Ventil aufweist, das die erste Messleitung mit der vierten Leitung verbindet. Die erste Messleitung mündet über dieses sechste Ventil dann in die vierte Leitung, über die dann die erste Messleitung wieder in Verbindung mit dem zweiten Pumpanschluss, oder über das fünfte Ventil mit dem ersten Pumpanschluss gebracht werden kann.

Weiterhin weist die Bioreaktorreinigungsanlage ein siebtes Ventil auf, das die erste Messleitung mit dem Säuretank und/oder Sammeltank verbindet. Vorzugsweise verbindet das siebte Ventil die erste Messleitung mit dem Säuretank und/oder dem Sammeltank über das zweite Säuretankventil bzw. das zweite Sammeltankventil. Das siebte Ventil verbindet folglich die Messleitung mit dem Säuretank über das zweite Säuretankventil und das siebte Ventil verbindet die erste Messleitung mit dem Sammeltank über das zweite Sammeltankventil. Zwischen dem siebten Ventil und dem zweiten Säuretankventil bzw. zweiten Sammeltankventil mündet vorzugsweise eine Frischwasserleitung oder eine Leitung der Dosiereinheit in die diese Ventile verbindende Leitung. So ist es möglich, ausgehend von der Frischwasserleitung oder der Leitung der Dosiereinheit, über das siebte Ventil Flüssigkeit in die erste Messleitung und damit zur Messeinheit zu befördern, und über das zweite Säuretankventil dem Säuretank zuzuführen und über das zweite Sammeltankventil dem Sammeltank zuzuführen.

In einer weiteren bevorzugten Ausführungsform weist die Bioreaktorreinigungsanlage einen Füllstandsensor in der Messeinheit auf zum Erfassen einer in der Messeinheit aufgenommenen Flüssigkeitsmenge, wobei der Füllstandsensor mit der elektronischen Steuereinheit verbunden ist und an dieser ein Messfüllstand-Signal bereitstellt. Über den Füllstandsensor in der Messeinheit kann das Volumen des in der Messeinheit vorhandenen Flüssigkeit erfasst werden und ein entsprechendes Messfüllstand-Signal an der elektronischen Steuereinheit bereitgestellt werden. Basierend auf dem Signal können dann ein oder mehrere Folgehandlungen durchgeführt werden, wie beispielsweise das Durchführen einer Reinigungsoperation, das Pumpen von Flüssigkeit von einem Säuretank zu dem zweiten Absauganschluss, oder das Absaugen von Flüssigkeit aus dem Bioreaktor.

Ferner ist bevorzugt, dass die Bioreaktorreinigungsanlage einen ersten pH-Sensor zum Erfassen eines ersten pH-Werts einer dem Säuretank zugeführten Flüssigkeit aufweist, wobei der erste pH-Sensor mit der elektronischen Steuereinheit verbunden ist und an diese ein erstes pH-Signal bereitstellt. Über das erste pH-Signal kann ermittelt werden, ob die dem Säuretank zugeführte Flüssigkeit einen ausreichend niedrigen pH-Wert hat, um als Flüssigkeit zur chemischen Reinigung des Bioreaktors verwendet zu werden. Dies ist einerseits relevant, wenn über die Dosiereinheit die wässrige Säurelösung erzeugt wird und in dem Säuretank bereitgestellt wird, aber auch, um zu ermitteln, ob eine aus dem Bioreaktor abgesaugte Flüssigkeit einen ausreichenden pH-Wert hat, um ein zweites oder weiteres Mal zur Reinigung verwendet zu werden, oder ob diese abgesaugte Flüssigkeit neutralisiert und entsorgt werden muss.

Gemäß einer weiteren bevorzugten Ausführungsform weist die elektronische Steuereinheit Codemittel auf, die, wenn auf der elektronischen Steuereinheit ausgeführt, diese veranlassen, ein Reinigungsverfahren mit einem oder mehreren Reinigungsschritten zum Reinigen und Warten des Bioreaktors durchzuführen.

In einem zweiten Aspekt löst die Erfindung die eingangs genannte Aufgabe durch ein Computerprogrammprodukt, aufweisend Codemittel, die, wenn auf einer elektronischen Steuereinheit einer Bioreaktorreinigungsanlage ausgeführt, diese veranlassen, ein Reinigungsverfahren mit einem oder mehreren Reinigungsschritten zum Reinigen und Warten des Bioreaktors mittels einer Bioreaktorreinigungsanlage gemäß dem ersten Aspekt der Erfindung durchzuführen, wobei das Reinigungsverfahren umfasst: Absaugen von Flüssigkeit aus dem Bioreaktor über einen zweiten Absauganschluss; und Pumpen der abgesaugten Flüssigkeit in einen Sammeltank.

Es soll verstanden werden, dass das Reinigungsverfahren, welches durch die Bioreaktorreinigungsanlage ausgeführt wird, wenn das Computerprogrammprodukt auf der Steuereinheit ausgeführt wird, auch eigenständig als Reinigungsverfahren hierin offenbart ist und beansprucht werden kann. Demnach ist hier auch ein Reinigungsverfahren mit einem oder mehreren Reinigungsschritten zum Reinigen und Warten des Bioreaktors mittels einer Bioreaktorreinigungsanlage gemäß dem ersten Aspekt der Erfindung offenbart, welches umfasst: Absaugen von Flüssigkeit aus dem Bioreaktor über einen zweiten Absauganschluss; und Pumpen der abgesaugten Flüssigkeit in einen Sammeltank.

Der zweite Absauganschluss ist vorzugsweise ein Anschluss für einen 2-Zoll-Schlauch eines Bioreaktors, der über eine Camlock-Kupplung mit der Bioreaktorreinigungsanlage gekoppelt werden kann. In einem Bioreaktor, insbesondere in dem Feststofftank und dort in dem Filterkorb, bildet sich ein Filterkuchen, der vor Durchführung von weiteren Reinigungsschritten zunächst abgesaugt werden sollte. Dies wird in dem Schritt des Absaugens von Flüssigkeit aus dem Bioreaktor umgesetzt. Diese Flüssigkeit wird in den Sammeltank gepumpt. Hierzu wird die Pumpe vorzugsweise von der elektronischen Steuereinheit angesteuert. Ebenso können ein oder mehrere Ventile angesteuert werden, um eine fluidleitende Verbindung zwischen dem zweiten Absauganschluss, der Pumpe und dem Sammeltank herzustellen.

Weiterhin ist bevorzugt, dass das Reinigungsverfahren umfasst: Einlesen von Daten von dem Bioreaktor über eine Datenverbindung zwischen dem Bioreaktor und der Bioreaktorreinigungsanlage. Die Bioreaktorreinigungsanlage hat zu diesem Zweck vorzugsweise einen 7-poligen Anschluss und kann aus dem Bioreaktor Daten auslesen wie z. B. ein letztes Wartungsintervall, Füllstände, Fehlerprotokolle und dergleichen. Das Ausführen von einem oder mehreren Reinigungsschritten des Reinigungsverfahrens kann in Abhängigkeit von diesen ausgelesenen Daten erfolgen. Hierdurch kann bevorzugt eine zustandsbezogene Wartung des Bioreaktors durchgeführt werden. Wird beispielsweise festgestellt, dass die letzte Reinigung einen längeren Zeitraum her ist, können sechs anstatt vier Absaugzyklen durchgeführt werden. Die Bioreaktorreinigungsanlage kann auch dazu ausgebildet sein, je nachdem, welche Schritte im Rahmen der Wartung sinnvoll und erforderlich sind, auf einem Display einem Bediener anzuzeigen, welche Schritte er/sie manuell ausführen soll. Solche Schritte können auch Schritte sein, die nicht notwendigerweise zur Reinigung allein dienen, sondern auch originär Wartungsschritte sein, wie z.B. der Austausch bestimmter Verschleißteile. Da der Bediener zu diesen Schritten im Rahmen der bevorzugten Ausführungsform aufgefordert wird, wird auch das Ergebnis angefordert. Es wird also ein Reinigungsergebnis dargestellt und vorzugsweise an den Bioreaktor übermittelt, vorzugsweise jedenfalls dann, wenn eine störungsfreie Reinigung/Wartung vorliegt. Vorzugsweise wird ein Wartungsprotokoll erstellt, insbesondere in der Bioreaktorreinigungsanlage, das alle oder einige Schritte und vorzugsweise die Bewertung durch den Bediener darstellt. Das Wartungsprotokoll wird vorzugsweise von der Bioreaktorreinigungsanlage ausgewertet und falls ein Schritt von dem Bediener und/oder automatisiert als "nicht ok" bewertet wurde, ist das Wartungsergebnis insgesamt "nicht ok". So kann genau protokolliert werden, was gemacht wurde und was nicht.

Weiterhin ist bevorzugt, dass das Reinigungsverfahren umfasst: Einfüllen von Frischwasser über den zweiten Absauganschluss in den Bioreaktor, und Warten einer vorbestimmten ersten Zeit. Das Einfüllen von Frischwasser über den zweiten Absauganschluss, der vorzugsweise mit einer Leitung des Bioreaktors verbunden ist, die unmittelbar in den Filterkorb mündet, dient dazu, einen dort vorhandenen Filterkuchen aufzuweichen und aufzuschwemmen, sodass anschließend eine bessere Absaugung stattfinden kann. Vorzugsweise wird dieser Schritt vor dem obigen Schritt des Absaugens von Flüssigkeit aus dem Bioreaktor durchgeführt. Die Schritte von Absaugen über den zweiten Absauganschluss sowie Einfüllen von Frischwasser über den zweiten Absauganschluss können auch zyklisch in zwei, drei, vier, fünf oder sechs Zyklen durchgeführt werden, um den Filterkorb möglichst weit abzusaugen. Die vorbestimmte erste Zeit kann mehrere Sekunden bis mehrere Minuten dauern, liegt aber vorzugsweise in einem Bereich von 20 Sekunden bis 5 Minuten.

Alternativ zu einer Datenverbindung mit dem Bioreaktor kann die Bioreaktorreinigungsanlage auch Daten von einer Prozessleitung des Bahnhofs, oder Zentral beispielsweise von einem Cloud-Dienst, dem Hersteller des Bioreaktors, einer Flottenleitung oder eines anderen Dienstleisters empfangen.

Die Bioreaktorreinigungsanlage kann aber nicht nur Daten aus dem Bioreaktor einlesen, sondern vorzugsweise auch Ventile des Bioreaktors schalten. Dies erfolgt vorzugsweise dadurch, dass die elektronische Steuereinheit entsprechende Signale an der elektronischen Schnittstelle (vorzugsweise 7-polig) bereitstellt. Hierdurch wird es möglich, nicht nur die von außen zugänglichen Leitungen und Tanks zu reinigen, sondern auch Leitungen im Innern des Bioreaktors, die erst durch Schalten von einem oder mehreren Ventilen des Bioreaktors zugänglich werden. Die elektronische Steuereinheit ist vorzugsweise dazu ausgebildet, ein oder mehrere auf das Schalten von einem oder mehreren Ventilen des Bioreaktors gerichteten Schaltsignalen an dem Bioreaktor bereitzustellen. Die elektronische Steuereinheit ist weiterhin vorzugsweise dazu eingerichtet, ein Reinigen von Leitungen des Bioreaktors zu veranlassen, vorzugsweise im Rahmen des Reinigungsverfahrens für den Bioreaktor.

Ferner umfasst das Reinigungsverfahren vorzugsweise: Zuführen einer Flüssigkeit zum Spülanschluss zum Zuführen der Flüssigkeit zu einer Reinigungsdüse des Bioreaktors. Das Zuführen von Flüssigkeit zum Spülanschluss wird vorzugsweise unter Druck ausgeführt. Hierzu wird vorzugsweise von der elektronischen Steuereinheit eine Hochdruckpumpe angesteuert, sodass die Flüssigkeit unter erhöhtem Druck der Reinigungsdüse zugeführt wird. Dies soll dazu dienen, den Filterkorb mechanisch abzureinigen. Die Reinigungsdüse ist üblicherweise fest in dem Bioreaktor verbaut und kann beispielsweise als Hohlkegeldüse oder Vollstrahldüse ausgebildet sein. Durch den hohen Druck wird der Filterkuchen im Filterkorb abgefräst und aufgeschwemmt, sodass die Partikel im Nachgang abgesaugt werden können. Daher schließt sich an diesen Schritt vorzugsweise ein Schritt des Absaugens von Flüssigkeit aus dem Bioreaktor über den zweiten Absauganschluss an. Die dann abgesaugte Flüssigkeit wird vorzugsweise in den Sammeltank gepumpt.

Ferner ist bevorzugt, dass das Verfahren umfasst: Absaugen von Flüssigkeit aus dem Bioreaktor über einen ersten Absauganschluss, und Pumpen der abgesaugten Flüssigkeit in den Sammeltank bzw. den Kanalabfluss oder in einen Säuretank. Der erste Absauganschluss der Bioreaktorreinigungsanlage wird vorzugsweise mit einem 1-Zoll-Anschluss des Bioreaktors verbunden, vorzugsweise wiederum über eine Camlock-Kupplung, der in den Flüssigkeitstank des Bioreaktors mündet. Flüssigkeit, die durch den Filterkorb im Bioreaktor geflossen ist, gelangt in den Flüssigkeitstank und kann über den ersten Absauganschluss abgesaugt werden. Je nachdem, um was für eine Flüssigkeit es sich handelt, wird diese entweder dem Sammeltank oder einem Säuretank zugeführt. Ist beispielsweise vorher eine chemische Reinigung mit einer wässrigen Säurelösung durchgeführt worden, wird die abgesaugte Flüssigkeit vorzugsweise dem Säuretank zugeführt. Handelt es sich um einfaches Wasser, das mit Partikeln beladen ist, beispielsweise Flüssigkeit, die aus einer vorhergehenden Reinigung mittels der Reinigungsdüse stammt, wird die so abgesaugte Flüssigkeit vorzugsweise dem Sammeltank zugeführt.

In einer weiteren bevorzugten Ausführungsform des Computerprogrammprodukts umfasst das Reinigungsverfahren: Einfüllen einer wässrigen Säurelösung über den ersten Absauganschluss und/oder den zweiten Absauganschluss in dem Bioreaktor und Warten einer vorbestimmten zweiten Zeit. Wässrige Säurelösung wird in den Bioreaktor eingeführt, um diesen von Kalkablagerungen zu befreien. Dies kann einerseits am Filterkorb notwendig sein, andererseits aber auch in Leitungen des Bioreaktors. Um Leitungen des Bioreaktors von Kalk zu reinigen, ist ein Zirkulieren der wässrigen Säurelösung in dem Bioreaktor erforderlich. Um dies zu bewirken, umfasst das Verfahren vorzugsweise: Einleiten von Druckluft in den Bioreaktor über den ersten Absauganschluss. Auf diese Weise wird die wässrige Säurelösung in Leitungen des Bioreaktors gedrückt, sodass diese von Kalkablagerungen befreit werden können. Auch diese Schritte können mehrfach wiederholt werden. An das Einfüllen der wässrigen Säurelösung schließt sich vorzugsweise nach dem Abwarten der vorbestimmten zweiten Zeit ein jeweiliges Absaugen über den ersten bzw. zweiten Absauganschluss an. Die Zeitspanne der vorbestimmten zweiten Zeit kann etwas länger sein als die Zeitspanne der vorbestimmten ersten Zeit, um der wässrigen Säurelösung ausreichend Zeit zu geben, die Kalkablagerungen aufzulösen. Die vorbestimmte zweite Zeit kann also mehrere Minuten betragen.

Über ein solches Zirkulieren kann nicht nur ein Bioreaktor gereinigt werden, sondern es ist auch möglich Toilettentanks und deren Leitungen in Zugwaggons zu reinigen. Hierzu kann die Bioreaktorreinigungsanlage an dafür vorgesehene Anschlüsse an dem jeweiligen Waggon angeschlossen werden. Je nach Aufbau des Waggons kann es aber auch denkbar und bevorzugt sein, dass die Bioreaktorreinigungsanlage 1 bestimmte Ventile im Waggon schaltet, um die Toilettentanks und deren Leitungen zu reinigen. Dies kann beispielsweise mittels der elektronischen Schnittstelle 32 erfolgen, über die die elektronische Steuereinheit 44 entsprechende Signale bereitstellen kann.

Vorzugsweise umfasst das Reinigungsverfahren ferner: Einfüllen einer wässrigen Säurelösung über den zweiten Absauganschluss in den Bioreaktor und gleichzeitig Absaugen von Flüssigkeit aus dem Bioreaktor über den ersten Absauganschluss. Auf diese Weise kann wässrige Säurelösung durch den Bioreaktor zirkuliert werden. Sie wird über den zweiten Absauganschluss bereitgestellt, dann von dort - bei korrekter Verbindung mit dem Bioreaktor - in den Filterkorb eingebracht, läuft durch diesen hindurch in den Flüssigkeitsreaktor, und wird aus diesem heraus in die Bioreaktorreinigungsanlage abgesaugt. Dort kann die wässrige Säurelösung direkt wieder zum zweiten Absauganschluss gepumpt werden, ohne in den Säuretank geleitet zu werden.

Soll die wässrige Säurelösung neutralisiert werden, kann dies auch im Bioreaktor selbst erfolgen. Zu diesem Zweck wird die wässrige Säurelösung wie beschrieben durch den Bioreaktor zirkuliert, und dann in der Bioreaktorreinigungsanlage zur Dosiereinheit bzw. durch diese hindurch gefördert, wobei dann ein entsprechendes Volumen Base zudosiert wird, um die wässrige Säurelösung zu neutralisieren. Wird die wässrige Säurelösung im Bioreaktor und nicht im Säuretank neutralisiert, kann die neutralisierte Flüssigkeit nach Abschluss der Neutralisation aus dem Bioreaktor abgesaugt und entweder dem Sammeltank oder direkt einem Kanalabfluss zugeführt werden, um diese zu entsorgen.

Weiterhin ist bevorzugt, dass das Reinigungsverfahren umfasst: Abmessen eines vorbestimmten Flüssigkeitsvolumens in einer Messeinheit; Zuführen eines vorbestimmten Flüssigkeitsvolumens in den Bioreaktor über den zweiten Absauganschluss; Warten einer vorbestimmten dritten Zeit; Absaugen von Flüssigkeit aus dem Bioreaktor über den ersten Absauganschluss; Zuführen der abgesaugten Flüssigkeit zu der Messeinheit; und Messen des Volumens der abgesaugten Flüssigkeit. Hierdurch kann eine Durchlässigkeit des Bioreaktors getestet werden. Das Volumen der abgesaugten Flüssigkeit, das gemessen wird, sollte idealerweise dem des vorbestimmten zugeführten Flüssigkeitsvolumens entsprechen. Herrscht hier ein Unterschied, der einen vorbestimmten Schwellwert überschreitet, zeigt dies eine mangelnde Durchlässigkeit des Bioreaktors an. Für diesen Fall sollte eine weitere Reinigung durchgeführt werden. Es kann auch vorgesehen sein, dass, sofern die Messung in der Messeinheit ergibt, dass der Schwellwert nicht unterschritten wird, über eine Mensch-Maschine-Schnittstelle ein Signal ausgegeben wird, welches angibt, dass die Reinigung erfolgreich war. Anderenfalls kann ein Signal ausgegeben werden, welches angibt, dass die Reinigung nicht erfolgreich war.

Vorzugsweise ist weiterhin vorgesehen, dass das Reinigungsverfahren umfasst: Reinigen eines Hygienisierungseinheit des Bioreaktors. Das Reinigen der Hygienisierungseinheit kann gleiche oder ähnliche Schritte umfassen, wie das Reinigen des Bioreaktors. Vorzugsweise wird zunächst der Spülanschluss mit dem Bioreaktor und/oder der Hygienisierungseinheit verbunden, falls diese einen eigenen Hochdruckanschluss mit Reinigungsdüse hat. Falls nicht, wird diese Ausrüstung vorzugsweise manuell an der Hygienisierungseinheit angebracht und mit dem Spülanschluss der Bioreaktorreinigungsanlage verbunden. Sind an der Hygienisierungseinheit separate Ablaufventile vorgesehen, müssen diese geöffnet werden. Für den Fall, dass die Hygienisierungseinheit über den Bioreaktor entleert werden kann, erfolgt dies in aller Regel über den Absauganschluss der Bioreaktorreinigungsanlage, der allerdings im Rahmen der Reinigung der Hygienisierungseinheit auch an einen separaten Anschluss der Hygienisierungseinheit angeschlossen werden kann. Das Reinigen der Hygienisierungseinheit umfasst dann: Zuführen von Flüssigkeit zum Spülanschluss zum Zuführen der Flüssigkeit zu einer Reinigungsdüse der Hygienisierungseinheit. Ferner umfasst das Reinigen der Hygienisierungseinheit vorzugsweise: Absaugen von Flüssigkeit aus der Hygienisierungseinheit über den ersten Absauganschluss der Bioreaktorreinigungseinheit oder einen dritten dafür vorgesehenen Absauganschluss. Das Zuführen und das Absaugen wird vorzugsweise zeitgleich ausgeführt, sodass ein Hochdruckstrahl der Reinigungsdüse Flächen der Hygienisierungseinheit ungehindert beaufschlagen kann. Wenn ausreichend mit der Düse gereinigt wurde und/oder eine vorbestimmte Zeit erreicht wurde, wird das Zuführen von Flüssigkeit zum Spülanschluss gestoppt und weiterhin Flüssigkeit aus der Hygienisierungseinheit abgesaugt, vorzugsweise für wenigstens einen vorbestimmten Zeitraum. Anschließend können gegebenenfalls Ventile geschlossen und Schläuche wieder getrennt werden.

Ausführungsformen der Erfindung werden nun nachfolgend anhand der Zeichnungen beschrieben. Diese sollen die Ausführungsformen nicht notwendigerweise maßstäblich darstellen, vielmehr sind die Zeichnungen, wenn dies zur Erläuterung dienlich ist, in schematisierter und/oder leicht verzerrter Form ausgeführt. Im Hinblick auf Ergänzungen der aus den Zeichnungen unmittelbar erkennbaren Lehren wird auf den einschlägigen Stand der Technik verwiesen. Dabei ist zu berücksichtigen, dass vielfältige Modifikationen und Änderungen betreffend die Form und das Detail einer Ausführungsform vorgenommen werden können, ohne von der allgemeinen Idee der Erfindung abzuweichen. Die in der Beschreibung, in den Zeichnungen sowie in den Ansprüchen offenbarten Merkmale der Erfindung können sowohl einzeln als auch in beliebiger Kombination für die Weiterbildung der Erfindung wesentlich sein. Zudem fallen in den Rahmen der Erfindung alle Kombinationen aus zumindest zwei der in der Beschreibung, den Zeichnungen und/oder den Ansprüchen offenbarten Merkmale. Die allgemeine Idee der Erfindung ist nicht beschränkt auf die exakte Form oder das Detail der im Folgenden gezeigten und beschriebenen bevorzugten Ausführungsformen oder beschränkt auf einen Gegenstand, der eingeschränkt wäre im Vergleich zu dem in den Ansprüchen beanspruchten Gegenstand. Bei angegebenen Bemessungsbereichen sollen auch innerhalb der genannten Grenzen liegende Werte als Grenzwerte offenbart und beliebig einsetzbar und beanspruchbar sein. Der Einfachheit halber sind nachfolgend für identische oder ähnliche Teile oder Teile mit identischer oder ähnlicher Funktion gleiche Bezugszeichen verwendet.

Weitere Vorteile, Merkmale und Einzelheiten der Erfindung ergeben sich aus der nachfolgenden Beschreibung der bevorzugten Ausführungsformen sowie anhand der Zeichnungen; diese zeigen in:
- Figur 1: eine schematische Darstellung der Bioreaktorreinigungsanlage im Zusammenhang mit einem Bioreaktor sowie weiteren Elementen;
- Figur 2: eine schematische Seitenansicht der Bioreaktorreinigungsanlage, teilweise freigeschnitten;
- Figur 3: einen Schaltplan der Bioreaktorreinigungsanlage;
- Figur 4: eine erste Ausführungsform eines Reinigungsverfahrens; und in
- Figur 5: eine zweite Ausführungsform eines Reinigungsverfahrens.

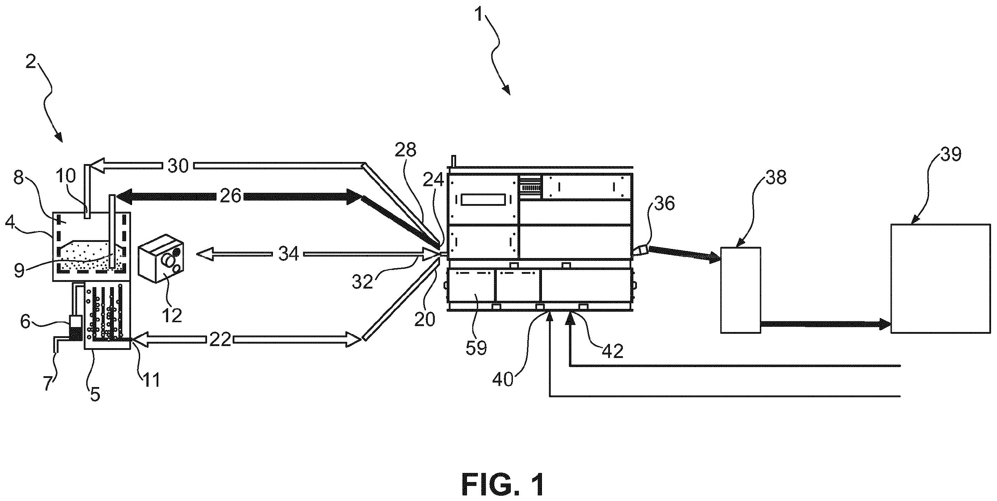

Eine Bioreaktorreinigungsanlage 1 kann als mobile Bioreaktorreinigungsanlage ausgebildet sein, wie in Fig. 1 gezeigt, oder auch als stationäre Bioreaktorreinigungsanlage. Eine mobile Bioreaktorreinigungsanlage kann typischerweise zu einem Zug, in dem ein Bioreaktor 2 angeordnet ist, verbracht werden. Bioreaktor 2 in Zügen sind im Allgemeinen bekannt und werden hier nicht weiter im Detail beschrieben. In Fig. 1 ist beispielhaft ein vertikal ausgerichteter Bioreaktor 2 gezeigt, mit einem Feststofftank 4, einem Flüssigkeitstank 5 sowie einer Hygienisierungseinheit 6, der einen Auslass 7 aufweist, zum Ablassen von Flüssigkeit. In dem Feststofftank 4 ist ein Filterkorb 8 vorgesehen, in den sowohl ein 2-Zoll-Schlauch 9 bodennah endet, als auch eine Reinigungsdüse 10, um dem Feststofftank 4 Wasser unter Hochdruck zuzuführen, um einen in dem Filterkorb 8 aufgebauten Filterkuchen abzureinigen. An dem Flüssigkeitstank 5 ist ferner ein 1-Zoll-Anschluss 11 ausgebildet, um Flüssigkeit aus dem Flüssigkeitstank 5 abzusaugen oder diesem zuzuführen. Ferner umfasst der Bioreaktor 2 eine Steuerung 12, die beispielsweise Sensoren des Bioreaktors 2 auslesen kann.

Die Bioreaktorreinigungsanlage 1 weist Anschlüsse auf, über die diese mit dem Bioreaktor 2 verbindbar ist. Um beispielsweise Flüssigkeit aus dem Bioreaktor 2 abzusaugen, weist die Bioreaktorreinigungsanlage 1 einen ersten Absauganschluss 20 auf, der über eine erste Absaugleitung 22 mit dem 1-Zoll-Anschluss 11 des Flüssigkeitstanks 5 des Bioreaktors 2 verbindbar ist. Ferner weist die Bioreaktorreinigungsanlage 1 einen zweiten Absauganschluss 24 auf, der über eine zweite Absaugleitung 26 mit dem 2-Zoll-Schlauch 9 des Bioreaktors 2 verbindbar ist, um über diesen, Flüssigkeit aus dem Bioreaktor 2, genauer gesagt den Feststofftank 4, abzusaugen, um so dort gebildeten Filterkuchen zu entfernen. Über den 2-Zoll-Schlauch 9 kann aber auch zu Reinigungszwecken Flüssigkeit in den Bioreaktor 2 gegeben werden, wie dies weiter unten noch genauer beschrieben werden wird

Die Bioreaktorreinigungsanlage 1 weist darüber hinaus einen Hochdruckanschluss 28 auf, der über einen Hochdruckschlauch 30 mit der Reinigungsdüse 10 verbindbar ist, sowie einen elektronischen Steueranschluss 32, der über eine Signalleitung 34 mit der Steuerung 12 des Bioreaktors 2 verbindbar ist.

Die Bioreaktorreinigungsanlage 1 weist ferner einen Entsorgungsanschluss 36 auf, über den die Bioreaktorreinigungsanlage 1 mit einem externen Tank 38 oder einer Kanalisation verbindbar ist, der mit einer externen Vakuumquelle 39 verbunden ist, um so Flüssigkeit aus der Bioreaktorreinigungsanlage 1 abzusaugen. Eingangsseitig weist die Bioreaktorreinigungsanlage 1 einen Spannungsanschluss 40 sowie einen Frischwasseranschluss 42 auf.

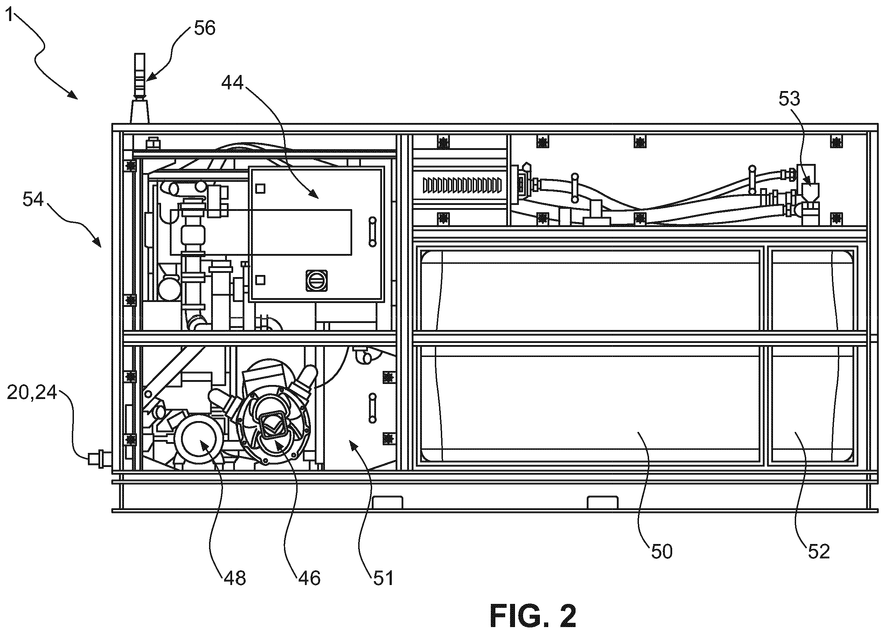

Im Inneren der Bioreaktorreinigungsanlage 1 (Fig. 2) weist diese eine elektronische Steuereinheit 44 auf, die einen Speicher mit Programmcode sowie einen Prozessor zum Ausführen des Programmcodes aufweist. Die elektronische Steuereinheit 44 steuert verschiedene Funktionen der Bioreaktorreinigungsanlage 1, wie sich insbesondere aus der weiteren Beschreibung ergibt. Beispielsweise steuert die elektronische Steuereinheit 44 eine Pumpe 46 sowie eine Hochdruckpumpe 48. Die Pumpe 46 kann sowohl dazu verwendet werden, ein Vakuum an dem ersten Absauganschluss 20 oder dem zweiten Absauganschluss 24 bereitzustellen, als auch Flüssigkeit zu dem zweiten Absauganschluss 24 zu pumpen. Die Hochdruckpumpe 48 dient dazu, eine Flüssigkeit mit Hochdruck an dem Hochdruckanschluss 28 bereitzustellen. Ferner sind im Inneren der Bioreaktorreinigungsanlage 1 ein Sammeltank 50 und ein Säuretank 52 vorgesehen, wobei für den Sammeltank 50 ein erster Füllstandssensor 51 und für den Säuretank 52 ein zweiter Füllstandssensor 53 vorgesehen sind. An der in Fig. 2 linken Seite der Bioreaktorreinigungsanlage 1 ist eine Mensch-Maschine-Schnittstelle 54 angeordnet, die beispielsweise ein Touch-Display umfasst. Über diese Mensch-Maschine-Schnittstelle 54 ist beispielsweise die elektronische Steuereinheit 44 bedienbar und beispielsweise können Parameter oder dergleichen eingegeben werden. Auch kann über die Mensch-Maschine-Schnittstelle 54 ein Reinigungsprogramm bzw. ein genauer Ablauf eines Reinigungsverfahrens gewählt werden. An einer Oberseite der Bioreaktorreinigungsanlage 1 ist optional eine Warnleuchte 56 vorgesehen, die dazu ausgebildet ist, Licht in verschiedenen Farben zu emittieren, um so einen Status, einen Fehler oder dergleichen der Bioreaktorreinigungsanlage 1 anzuzeigen.

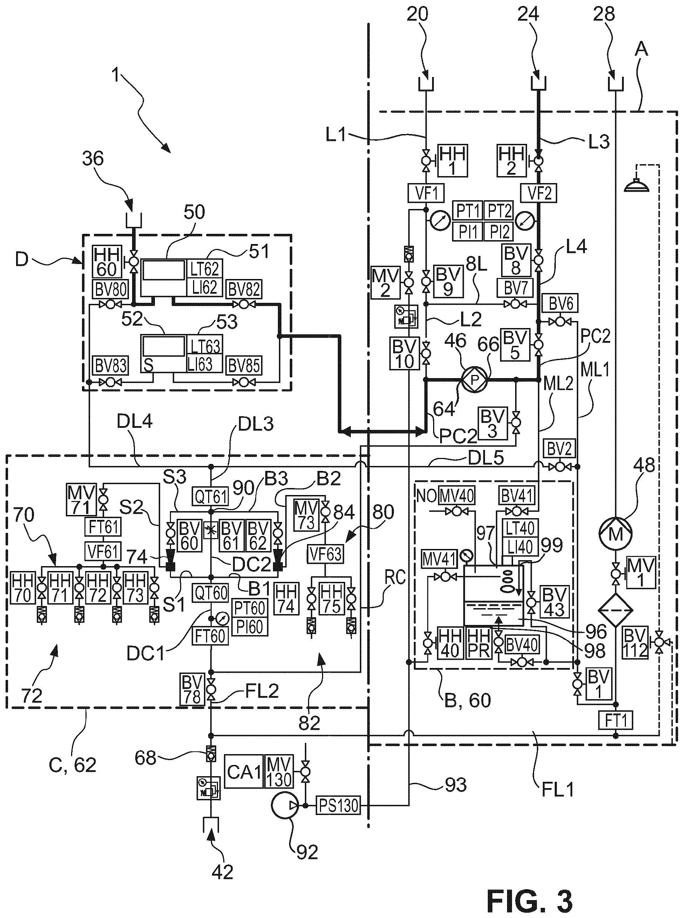

Fig. 3 zeigt nun ein vollständiges Layout bzw. einen vollständigen Schaltplan der mobilen Bioreaktorreinigungsanlage 1, in der die Pumpe 46 sowie der erste Absauganschluss 20, der zweite Absauganschluss 24, der Spülanschluss 28, der Entsorgungsanschluss 36 und der Frischwasseranschluss 42 eingezeichnet sind. Ferner ist die Hochdruckpumpe 48 gezeigt. Nicht in Fig. 3 gezeigt sind die elektronischen Verbindungen sowie die elektronische Steuereinheit 44. Es soll aber verstanden werden, dass die elektronische Steuereinheit 44 tatsächlich mit der Pumpe 46 sowie der Hochdruckpumpe 48 verbunden ist, sowie auch mit einigen oder allen der weiteren im Folgenden beschriebenen Ventilen und Sensoren. Der Schaltplan bzw. das Layout einer stationären Bioreaktorreinigungsanlage kann in Details leicht abweichen, die Funktionalität ist aber im Wesentlichen dieselbe und auch stationäre Bioreaktorreinigungsanlagen sind von der Erfindung mit umfasst.

In Fig. 3 ist die Bioreaktorreinigungsanlage 1 in vier Systeme untergliedert dargestellt, nämlich ein System A, das die Pumpe 46 sowie die Hochdruckpumpe 48 umfasst, und das auch die ersten und zweiten Absauganschlüsse 20, 24, sowie den Spülanschluss 28 umfasst. Ein System B ist innerhalb des Systems A gezeigt und umfasst eine Messeinheit 60, die noch genauer beschrieben werden wird. System C umfasst eine Säuredosiereinheit 62 und System D umfasst den Sammeltank 50, den Säuretank 52, die entsprechend zugeordneten Füllstandssensoren 51, 53 und den Entsorgungsanschluss 36.

Die Pumpe 46 hat einen ersten Pumpanschluss 64 und einen zweiten Pumpanschluss 66. Die Pumpe 46 ist vorzugsweise als Drehkolbenpumpe ausgebildet und kann sowohl Flüssigkeit vom ersten Pumpanschluss 64 zum zweiten Pumpanschluss 66 pumpen als auch umgekehrt vom zweiten Pumpanschluss 66 zum ersten Pumpanschluss 64.

Von dem ersten Absauganschluss 20 aus verläuft eine erste Leitung L1 in Richtung des ersten Pumpanschlusses 64. Die erste Leitung L1 ist mit einem ersten Ventil BV9 verbunden, welches seinerseits mit einer zweiten Leitung L2 verbunden ist. Das erste Ventil BV9 ist als elektrisch schaltbares Kugelventil ausgebildet und kann durch die elektronische Steuereinheit 44 gesteuert werden. Auch andere Ventiltypen, wie beispielsweise Schaltventile, sind bevorzugt. Wichtig im Rahmen der Erfindung ist nun, dass einige der Ventile elektrisch durch die elektronische Steuereinheit 44 schaltbar sind. Ein Kugelventil hat den Vorteil, dass der Fluss durch das Ventil stufenlos einstellbar ist. In die erste Leitung L1 ist zudem in der gezeigten Ausführungsform ein erstes Handventil HH1 eingesetzt, welches ein manuelles Öffnen und Schließen der ersten Leitung L1 ermöglicht. Zwischen dem ersten Ventil BV9 und dem ersten Handventil HH1 ist zudem ein erster kapazitiver Sensor VF1 vorgesehen, der die Anwesenheit von Flüssigkeit in der ersten Leitung L1 erfasst und ein die Anwesenheit von Flüssigkeit in der ersten Leitung L1 repräsentierendes Signal an der elektronischen Steuereinheit 44 bereitstellt.

Das zweite Ventil BV10 ist hier mit dem ersten Pumpanschluss 64 verbunden, genauer gesagt mit einer ersten Pumpleitung PL1, die von dem ersten Pumpanschluss 64 ausgeht. Die erste Pumpleitung PL1 verläuft mit Bezug auf Fig. 3 nach links zum System D.

Der zweite Absauganschluss 24 ist mit einer dritten Leitung L3 verbunden, die über ein drittes Ventil BV8 mit einer vierten Leitung L4 verbunden ist. Wiederum ist in die dritte Leitung L3 ein zweites Handventil HH2 eingesetzt, das ein manuelles Öffnen und Schlie-ßen der dritten Leitung L3 erlaubt. Zwischen das zweite Handventil HH2 und das dritte Ventil BV8 ist ein zweiter kapazitiver Sensor VF2 gesetzt, der auch dort die Anwesenheit von Flüssigkeit erfasst und ein entsprechendes die Anwesenheit von Flüssigkeit repräsentierendes Signal an der elektronischen Steuereinheit 44 bereitstellt. In der ersten Leitung L1 ist zudem ein erster Drucksensor PT1 und in der dritten Leitung L3 ein zweiter Drucksensor PT2 vorgesehen, die Druck in den ersten und dritten Leitungen L1, L3 erfassen und entsprechende erste und zweite Drucksignale an der elektronischen Steuereinheit 44 bereitstellen. Die vierte Leitung L4 ist über ein viertes Ventil BV5 mit dem zweiten Pumpanschluss 66 verbunden, genauer gesagt mit einer zweiten Pumpleitung PL2. Die zweite Pumpleitung PL2 verbindet den zweiten Pumpanschluss 66 mit dem vierten Ventil BV5.

Zwischen der zweiten Leitung L2 und der vierten Leitung L4 ist gemäß dem hier gezeigten Ausführungsbeispiel auch ein Bypass vorgesehen, nämlich in Form einer Bypass-Leitung BL, die durch ein fünftes Ventil BV7 verschließbar ist. Die Bypass-Leitung BL wird dazu verwendet, es zu ermöglichen, dass nicht nur der erste Absauganschluss 24 über die erste Leitung L1 und die zweite Leitung L2 mit dem ersten Pumpanschluss 64 verbindbar ist, sondern der erste Absauganschluss 20 ist über die erste Leitung L1, die Bypass-Leitung BL und die vierte Leitung L4 auch mit dem zweiten Pumpanschluss 66 verbindbar. In übereinstimmender Weise ist auch der zweite Absauganschluss 24 nicht nur über die dritte und vierte Leitung L3, L4 mit dem zweiten Pumpanschluss 66 verbindbar, sondern auch über die dritte Leitung L3, die Bypass-Leitung BL sowie die zweite Leitung L2 und die erste Pumpleitung PL1 mit dem ersten Pumpanschluss 64 verbindbar. Je nachdem, in welche Richtungen Flüssigkeiten gepumpt werden sollen, ist dies vorteilhaft.

Wird beispielsweise in einem ersten Reinigungsschritt Flüssigkeit aus dem Bioreaktor 2 abgesaugt, erfolgt dies über den zweiten Absauganschluss 24. Hierzu ist das zweite Handventil HH2 zu öffnen, gleichzeitig öffnet die elektronische Steuereinheit das dritte Ventil BV8 und das vierte Ventil BV4 und die Pumpe befördert die abgesaugte Flüssigkeit von dem zweiten Pumpanschluss 66 zum ersten Pumpanschluss 64 in die erste Pumpleitung PL1. Das zweite Ventil BV10 ist geschlossen und die Flüssigkeit strömt durch die erste Pumpleitung PL1 in Richtung des Systems D. Im System D ist der Sammeltank 50 über ein erstes Sammeltankventil BV82 mit der ersten Pumpleitung PL1 verbunden und der Säuretank 52 über ein erstes Säuretankventil 85 mit der ersten Pumpleitung PL1 verbunden. Um also die abgesaugte Flüssigkeit vom zweiten Absauganschluss 24 in den Sammeltank 50 zu befördern, öffnet die elektronische Steuereinheit 44 auch das erste Sammeltankventil 82. Soll nun zusätzlich über den ersten Absauganschluss 20 Flüssigkeit aus dem Flüssigkeitstank 5 des Bioreaktors 2 abgesaugt werden, muss das erste Handventil HH1 geöffnet werden. Die elektronische Steuereinheit 44 öffnet dann das erste Ventil BV9, das fünfte Ventil BV7 sowie das vierte Ventil BV5. Auf diese Weise ist der erste Absauganschluss 20 mit dem zweiten Pumpanschluss 66 verbunden. Die Pumpe 46 kann dann wiederum vom zweiten Pumpanschluss 66 zum ersten Pumpanschluss 64 pumpen und so die über den ersten Absauganschluss 20 abgesaugte Flüssigkeit über das erste Sammeltankventil 82 in den Sammeltank 50 befördern.

Das Entleeren des Sammeltanks 5 in den Entsorgungsbehälter 38 oder in die Kanalisation erfolgt über ein drittes Handventil HH50, das den Sammeltank 50 mit dem Entsorgungsanschluss 36 verbindet.

Insbesondere in System D existieren Unterschiede zwischen der hier gezeigten mobilen Bioreaktorreinigungsanlage 1 und einer stationären Bioreaktorreinigungsanlage. So kann zum Entleeren des Sammeltanks 50 und des Säuretanks 52 eine separate zusätzliche Pumpe vorgesehen sein, vorzugsweise in Form einer Doppelpumpe. Zudem ist vorzugsweise eine weitere Pumpe vorgesehen, wieder in Form einer Doppelpumpe, um den Säuretank 52 mit Säure zu füllen und um die Säure aus dem Säuretank in den jeweils angeschlossenen Bioreaktor zu füllen. Mittels der weiteren Pumpe kann in diesem Fall auch ein Zirkulieren der Flüssigkeit durch den Bioreaktor ausgeführt werden. Zudem kann eine stationäre Anlage einen zusätzlichen Anschluss zum Bereitstellen von Frischwasser für ein Catering im Waggon aufweisen sowie einen weiteren zusätzlichen Anschluss zum Bereitstellen von Frischwasser für eine Handwäsche und/oder Toilettenspülung. Eine Abzweigleitung für diesen zusätzlichen Anschluss zweigt vorzugsweise direkt vom Frischwasseranschluss 42 ab, sodass hier keine Kontamination stattfinden kann.

Mit der vierten Leitung L4 ist auch ein sechstes Ventil BV6 verbunden, das die vierte Leitung L4 mit einer ersten Messleitung ML1 verbindet. Die erste Messleitung ML1 führt einerseits zur Messeinheit 60, andererseits auch zu einem achten Ventil BV1, welches über einen ersten Durchflusssensor FT1 mit einer ersten Frischwasserleitung FL1 verbunden ist. Die erste Frischwasserleitung FL1 ist mit dem Frischwasseranschluss 42 über ein Rückschlagventil 68 verbunden und empfängt so von dem Frischwasseranschluss 42 Frischwasser. Soll beispielsweise über den zweiten Absauganschluss 24 Frischwasser in den Filterkorb 8 des Bioreaktors 2 gegeben werden, sind hierzu dann das achte Ventil BV1, das sechste Ventil BV6 und das dritte Ventil BV8 zu öffnen. Über den Frischwasseranschluss 42 wird Frischwasser bereits unter einem gewissen Druck bereitgestellt, und kann so direkt mit ausreichendem Druck in den Bioreaktor 2 geführt werden. Soll allerdings Frischwasser unter erhöhtem Druck der Reinigungsdüse 10 über den Spülanschluss 28 zugeführt werden, muss hierzu zunächst ein neuntes Ventil MV1 geöffnet werden, welches hier als Magnetventil ausgebildet ist. Das neunte Ventil MV1 verbindet die erste Frischwasserleitung FL1 stromabwärts des ersten Durchflusssensors FT1 mit der Hochdruckpumpe 48, die dann Frischwasser unter Hochdruck dem Spülanschluss 28 bereitstellen kann. Auch das neunte Ventil MV1 sowie die Hochdruckpumpe 48 werden von der elektronischen Steuereinheit 44 gesteuert.

Der Frischwasseranschluss 42 ist ferner über eine zweite Frischwasserleitung FL2 mit der Säuredosiereinheit 62 verbunden. Die Säuredosiereinheit 62 umfasst eine Mehrzahl an Säurekanister-Anschlüssen 70, sowie eine Mehrzahl an Basekanister-Anschlüssen 80. Die Säurekanister-Anschlüsse 70 sind mit Säurekanistern 72 verbunden, die Basekanister-Anschlüsse 80 mit Basekanistern 82. Die Säure- und Basekanister 72, 82 können ausgetauscht werden und sind beispielsweise am unteren Abschnitt der Bioreaktorreinigungsanlage 1 gelagert. Die zweite Frischwasserleitung FL2 führt zu einem zehnten Ventil BV78, und von dort zu einem zweiten Durchflusssensor FT60. Stromabwärts des zweiten Durchflusssensors FT60 sind ein dritter Drucksensor PT60 sowie ein erster pH-Sensor QT60 vorgesehen. Über den zweiten Durchflusssensor FT60, den dritten Drucksensor PT60 und den ersten pH-Sensor QT60 können Werte der in der ersten Dosierleitung DL1 vorhandenen Flüssigkeit erfasst werden. Stromabwärts des ersten pH-Sensors QT60 verzweigt sich die erste Dosierleitung DL1 in eine Säureleitung S1, eine zweite Dosierleitung DL2 sowie eine erste Baseleitung B1. Die erste Säureleitung S1 führt zu einem Säure-Dosierer 74, der hier als Säure-Ejektor ausgebildet ist und neben Flüssigkeit von der ersten Dosierleitung DL1 bzw. ersten Säureleitung S1 auch unverdünnt Säure über eine zweite Säureleitung S2 empfängt, die mit den Säurekanister-Anschlüssen 70 verbunden ist. In die zweite Säureleitung S2 ist ein elftes Ventil MV71 eingesetzt, welches als Magnetventil ausgebildet ist. Es dient dazu, die Säurekanister-Anschlüsse 70 gegenüber der zweiten Säureleitung S2 abzuriegeln. Stromabwärts des Säure-Dosierers 74 ist ein zwölftes Ventil BV60 vorgesehen, welches wiederum als Kugelventil ausgebildet ist und wiederum von der elektronischen Steuereinheit 44 gesteuert wird. Das zwölfte Ventil BV60 verbindet den Säure-Dosierer 74 mit einer dritten Säureleitung S3, die zu einem Mischer 90 führt. Der Mischer 90 kann beispielsweise einen statischen Mischer mit einem Mischelement umfassen.

Auf der anderen Seite verbindet die erste Baseleitung B1 die zweite Durchflussleitung DL2 mit einem Base-Dosierer 84, der hier als Base-Ejektor ausgebildet ist. Der Base-Dosierer 84 empfängt nicht nur Flüssigkeit über die erste Baseleitung B1, sondern auch unverdünnte Base über eine zweite Baseleitung B2. In die zweite Baseleitung B2 ist ein dreizehntes Ventil MV73 eingesetzt, welches als Magnetventil ausgebildet ist, und von der elektronischen Steuereinheit 44 gesteuert wird. das dreizehnte Ventil MV73 dient dazu, die Basekanister-Anschlüsse 80 gegenüber der zweiten Baseleitung B2 bzw. dem Base-Dosierer 84 abzuriegeln. Stromabwärts des Base-Dosierers 84 ist ein vierzehntes Ventil BV62 vorgesehen, welches wiederum als Kugelventil ausgebildet ist und von der elektronischen Steuereinheit 44 gesteuert wird. Das vierzehnte Ventil BV62 verbindet den Base-Dosierer 84 mit einer dritten Baseleitung B3, die auch in den Mischer 90 mündet. Über diese Anordnung kann also eine Flüssigkeit mit einem bestimmten pH-Wert in dem Mischer 90 erzeugt werden. Stromabwärts des Mischers 90 mündet dieser in eine dritte Dosierleitung DL3, in die ein zweiter pH-Sensor QT61 eingesetzt ist, der ein zweites pH-Signal an der elektronischen Steuereinheit 44 bereitstellt. Die dritte Dosierleitung DL3 verzweigt sich in eine vierte Dosierleitung DL4, die zum Säuretank 52 bzw. Sammeltank 55 führt, und eine fünfte Dosierleitung DL5, die zurück zum System A führt. Die vierte Dosierleitung DL4 ist mit einem zweiten Säuretankventil BV83 und einem zweiten Sammeltankventil BV80 verbunden, sodass Flüssigkeit aus der vierten Dosierleitung DL4 wahlweise über das zweite Säuretankventil BV83 und das zweite Sammeltankventil BV80 in den Säuretank 52 bzw. Sammeltank 50 geführt werden kann. Dies ist insbesondere dann wichtig, wenn eine wässrige Säurelösung in dem Säuretank 52 erzeugt werden soll. Zu diesem Zweck steuert die elektronische Steuereinheit 44 das zehnte Ventil BV78, das elfte Ventil MV71, das zwölfte Ventil BV60, das vierzehnte Ventil BV62 sowie das dreizehnte Ventil MV73 so, dass eine Flüssigkeit mit einem vorbestimmten gewünschten pH-Wert in der vierten Dosierleitung DL2 bereitgestellt werden und so in den Säuretank 52 gelangen kann.

Wässrige Säurelösung wird insbesondere dazu verwendet, den Bioreaktor 2 chemisch zu reinigen, um so Kalkablagerungen an diesem zu entfernen. Dazu muss wässrige Säurelösung aus dem Säuretank 52 zum ersten und/oder zweiten Absauganschluss 20, 24 geführt werden. Dies wird durch die Pumpe 46 bewirkt, die auf entsprechende Weise mit dem Säuretank 52 verbunden wird. Soll beispielsweise wässrige Säurelösung an dem ersten Absauganschluss 20 bereitgestellt werden, öffnet die elektronische Steuereinheit 44 das erste Säuretankventil BV85, das vierte Ventil BV5, das fünfte Ventil BV7 und das erste Ventil BV9. Wässrige Säurelösung wird dann über die erste Pumpleitung PL1, vom ersten Pumpanschluss 64 zum zweiten Pumpanschluss 66 und über die genannten Ventile bis hin zum ersten Absauganschluss 20 geführt. In entsprechender Weise kann eine wässrige Säurelösung auch an dem zweiten Absauganschluss 24 bereitgestellt werden, wobei dann in Abweichung zu dem Vorgenannten nicht das fünfte und erste Ventil BV7, BV9 geöffnet werden müssen, sondern stattdessen nur das dritte Ventil BV3.

Wurde eine wässrige Säurelösung zum Reinigen des Bioreaktors 2 verwendet, ist es erforderlich, diese wieder auch aus dem Bioreaktor 2 abzusaugen. Dies geschieht über den ersten Absauganschluss 20. Wird eine wässrige Säurelösung aus dem Bioreaktor 2 über den Absauganschluss 20 abgesaugt, werden hierzu das erste Ventil BV9, das fünfte Ventil BV7, das vierte Ventil BV5 sowie das erste Säuretankventil BV85 geöffnet. Die Pumpe 46 pumpt dann die wässrige Säurelösung vom zweiten Pumpanschluss 66 zum ersten Pumpanschluss 64, und infolgedessen in den Säuretank 52.

Soll die wässrige Säurelösung im Säuretank 52 neutralisiert werden, steuert die elektronische Steuereinheit 44 die Säuredosiereinheit 62 entsprechend, um eine Flüssigkeit bereitzustellen, die geeignet ist, die wässrige Säurelösung im Säuretank 52 zu neutralisieren. Es ist auch möglich, eine Durchlaufneutralisierung zu erreichen. Zu diesem Zweck wird wässrige Säurelösung aus dem Säuretank 52 über das erste Säuretankventil 85 mittels der Pumpe 46 abgesaugt, vom ersten Pumpanschluss 64 zum zweiten Pumpanschluss 66 gepumpt, und von dort über ein Rückflussventil BV3, welches die zweite Pumpleitung PL2 bzw. den zweiten Pumpanschluss 66 mit der Säuredosiereinheit 62 verbindet. Genauer gesagt führt eine Rückführleitung RL von dem Rückführventil BV3 zu der zweiten Frischwasserleitung FL2 und mündet in diese stromabwärts des zehnten Ventils BV78, aber stromaufwärts des ersten Durchflussmessers 60. Mittels des ersten pH-Sensors QT60 kann dann der pH-Wert der so rückgeführten wässrigen Säurelösung aus dem Säuretank 52 ermittelt werden und die Ventile BV60, BV62 sowie eine Drossel BV61 so gesteuert werden, dass der wässrigen Säurelösung, die über die Rückführleitung RL3 bereitgestellt wird, ausreichend Base zudosiert wird, um diese zu neutralisieren. Nachdem die Lösung in dem Säuretank 52 neutralisiert wurde, kann sie mittels der Pumpe 46 in den Sammeltank 50 befördert werden.

Um die Reinigung des Bioreaktors 2 mit wässriger Säurelösung zu verbessern, kann auch Luft in die über den ersten Absauganschluss 20 eingeführte wässrige Säurelösung eingeperlt werden. Dazu ist ein Kompressor 92 vorgesehen, der über eine Kompressorleitung 93 und ein Kompressorventil MV2 mit der ersten Leitung L1 verbunden ist und so Druckluft in die erste Leitung L1 einspeisen kann. Auch der Kompressor 92 sowie das erste Kompressorventil MV2 können über die elektronische Steuereinheit 44 gesteuert werden.

Die Messeinheit 60 ist nun wie folgt aufgebaut und mit den weiteren Elementen verbunden: Die Messeinheit 60 umfasst eine Messkammer 96 mit einem ersten Anschluss 97, einem zweiten Anschluss 98 und einem dritten Anschluss 99. Der erste Anschluss 97 ist über ein erstes Messventil BV41 mit der zweiten Pumpleitung PL2 verbunden, insbesondere über eine zweite Messleitung ML2. Der zweite Anschluss 98 ist über ein zweites Messventil BV40 mit der ersten Messleitung ML1 verbunden, und der dritte Anschluss 99 ist über ein drittes Messventil BV43 ebenfalls mit der ersten Messleitung ML1 verbunden. An der Messkammer 96 ist ferner ein Füllstandssensor LT40 vorgesehen, der mit der elektronischen Steuereinheit 44 verbunden ist und an dieser ein Messfüllstand-Signal bereitstellen kann. Die Messeinheit 60 wird dazu verwendet, die Durchlässigkeit des Bioreaktors 2 zu testen, nachdem eine Reinigung durchgeführt wurde. Zu diesem Zweck wird in der Messkammer 96 zunächst ein vorbestimmtes Volumen an Frischwasser abgemessen. Dies erfolgt vorzugsweise durch Öffnen des achten Ventils BV1 und des zweiten Messventils BV40. Auf diese Weise kann Frischwasser durch den zweiten Anschluss 98 in die Messkammer 96 strömen, so lange, bis ein vorbestimmtes Volumen erreicht ist, was mittels des Messfüllstand-Signals ermittelt wird.

Nachdem das vorbestimmte Volumen in der Messkammer 96 abgemessen wurde, kann dieses über den zweiten Absauganschluss 24 dem Bioreaktor 2 zugeführt werden. Zu diesem Zweck wird das erste Messventil BV41 geöffnet, die Flüssigkeit vom zweiten Pumpanschluss 66 zum ersten Pumpanschluss 64 gepumpt, dann weiter über das zweite Ventil BV10, die Bypass-Leitung BL, das fünfte Ventil BV7 sowie das dritte Ventil BV8 zum zweiten Absauganschluss 24. Anschließend wird eine vorbestimmte Zeit gewartet, bis die Flüssigkeit durch den Bioreaktor 2 gelaufen ist. Anschließend wird über den ersten Absauganschluss 20 aus dem Flüssigkeitstank 5 abgesaugt, indem das erste Ventil BV9 und das zweite Ventil BV10 geöffnet werden, die Flüssigkeit vom ersten Pumpanschluss 64 zum zweiten Pumpanschluss 66 gepumpt wird und dann über das vierte Ventil BV5, das sechste Ventil BV6 und das dritte Messventil BV43 in die Messkammer 96 eingebracht wird. Dort wird die abgesaugte Flüssigkeit wieder gemessen. Wenn die Volumendifferenz zwischen der zugeführten Flüssigkeit und der abgesaugten Flüssigkeit einen vorbestimmten Grenzwert nicht überschreitet, ist die Reinigung in Ordnung. Überschreitet die Volumendifferenz einen vorbestimmten Grenzwert, ist die Reinigung nicht in Ordnung und ein entsprechendes Warnsignal kann beispielsweise über die Warnleuchte 56 und/oder die Mensch-Maschine-Schnittstelle 54 ausgegeben werden. Der Vergleich, ob die Volumendifferenz den vorbestimmten Schwellwert überschreitet oder nicht, wird vorzugsweise von der elektronischen Steuereinheit 44 ausgeführt.

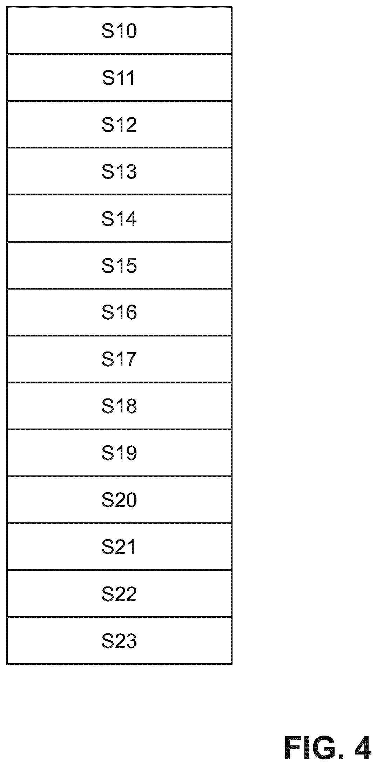

Anhand von Figur 4 wird nun ein erstes Ausführungsbeispiel eines Reinigungsverfahrens gemäß der Erfindung dargestellt. Das Reinigungsverfahren wird als solches hierin offenbart. Es soll aber verstanden werden, dass das Computerprogramm gemäß der Erfindung, welches auf der Steuereinheit 44 der Bioreaktorreinigungsanlage 1 gespeichert und/oder dort ausgeführt werden kann, die Bioreaktorreinigungsanlage 1 veranlasst, das Reinigungsverfahren durchzuführen. Das Reinigungsverfahren, welches in Figur 4 erläutert wird, kann zum Beispiel 28 Schritte umfassen, wobei das Verfahren auch mehr oder weniger Schritte umfassen kann. Das Reinigungsverfahren funktioniert im Grunde zyklisch und kann als mechanisches Reinigungsverfahren ausgeführt werden, in welchem keine Säure in den Bioreaktor 2 eingeführt wird, oder als chemisches Reinigungsverfahren, bei welchem Säure benutzt wird, um den Bioreaktor 2 zu reinigen.

In Schritt S10 wird zunächst über den zweiten Absauganschluss 24 Frischwasser von dem Frischwasseranschluss 42 in den Bioreaktor 2 eingeführt. Hierzu schaltet die elektronische Steuereinheit 44 die entsprechenden Ventile und steuert die Pumpe 46, wie vorstehend im Grunde beschrieben. In diesem ersten Schritt S10 werden vorzugsweise ca. 50 Liter Wasser in den Bioreaktor 2 eingebracht. Dies sollte ca. 1 Minute in Anspruch nehmen. In Schritt S11 wird dann über den zweiten Absauganschluss 24 Flüssigkeit aus dem Bioreaktor 2 abgesaugt und in den Sammeltank 50 gepumpt. Auch hierzu steuert die elektronische Steuereinheit 44 die entsprechenden Ventile und die Pumpe 46 an. Dies wird vorzugsweise so lange ausgeführt, bis der zweite kapazitive Sensor VF2 erfasst, dass keine Flüssigkeit mehr in der Leitung L3 vorhanden ist. Schritt S12 ist dann optional und in diesem wird Flüssigkeit aus dem Bioreaktor über den ersten Absauganschluss 20 abgesaugt. Dies ist nicht zwingend erforderlich, kann aber umgesetzt werden, um den Bioreaktor von dieser Flüssigkeit zu reinigen. Anschließend wird in Schritt S13 vorzugsweise wiederum Flüssigkeit, vorzugsweise Frischwasser, über den zweiten Absauganschluss 24 in den Bioreaktor 2 gegeben. Auch über den ersten Absauganschluss 20 kann in Schritt S15 Flüssigkeit in den Bioreaktor 2 gegeben werden. Dies dient beides zum Aufschwemmen von Feststoffen in dem Bioreaktor 2. In Schritt S16 wird dann vorzugsweise zusätzlich eine mechanische Reinigung durchgeführt, indem über den Spülanschluss 28 Frischwasser unter Hochdruck bereitgestellt wird. Hierzu steuert die elektronische Steuereinheit 44 die entsprechenden Ventile, nämlich insbesondere das neunte Ventil MV1 sowie die Hochdruckpumpe 48 an. Bei diesem Spülen mit Hochdruck soll vorzugsweise nur 40 % des Bioreaktor-Volumens mit Wasser gefüllt werden. Dies kann je nach Bioreaktor 2 einem Volumen von etwa 70 bis 100 Litern entsprechen.

In Schritt S17 wird dann wiederum über den zweiten Absauganschluss 24 Flüssigkeit aus dem Bioreaktor 2 abgesaugt, in Schritt S18 auch über den ersten Absauganschluss 20. Sowohl in Schritt S17 als auch in Schritt S18 wird vorzugsweise so lange abgesaugt, bis die ersten und zweiten kapazitiven Sensoren VF1, VF2 erfassen, dass keine Flüssigkeit mehr in der ersten bzw. der dritten Leitung L1, L3 vorhanden ist. Die Schritte S19 bis S23 sind dann vorzugsweise Wiederholungen der Schritte S14 bis S18 und können so oft wiederholt werden wie erforderlich, um eine ausreichende Reinigung zu erzielen. Es kann allerdings vorgesehen sein, dass in den nachfolgenden Schritten, in denen Flüssigkeit über den Spülanschluss 28 bereitgestellt wird, auch höhere Füllgrade des Bioreaktors 2 erlaubt werden, beispielsweise 60, 70 oder 80 %. Es kann auch vorgesehen sein, dass in Schritt S18 sowie in Schritt S23 zunächst nicht über den ersten Absauganschluss 20 abgesaugt wird, sondern diese Flüssigkeit in dem Bioreaktor 2 verbleibt. Erst in dem letzten Schritt, bevor das Verfahren beendet wird, wird über den ersten Absauganschluss 20 abgesaugt, um den Bioreaktor 2, nämlich insbesondere auch den Flüssigkeitstank 5, vollständig zu leeren.

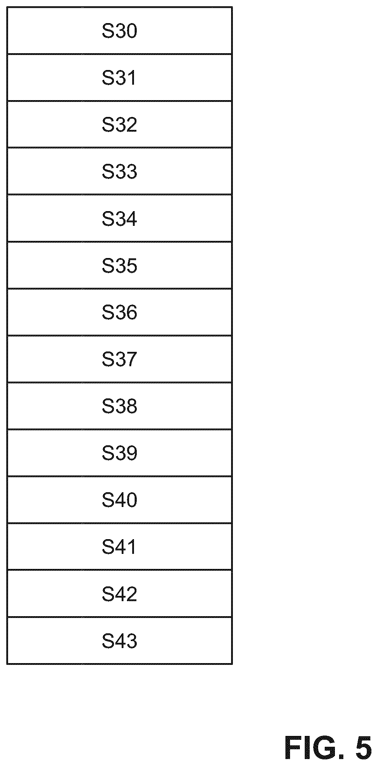

Figur 5 illustriert hingegen einen chemischen Reinigungsprozess und damit ein weiteres Ausführungsbeispiel des Reinigungsverfahrens. Es soll aber verstanden werden, dass das mechanische Reinigungsverfahren gemäß Figur 4 auch im Rahmen des Reinigungsverfahrens gemäß Figur 5 kombiniert werden kann. Zum Beispiel wird zunächst im Rahmen des Reinigungsverfahrens das mechanische Verfahren gemäß Figur 4 ausgeführt und im Anschluss das chemische Reinigungsverfahren gemäß Figur 5.

In Schritt S30 wird eine wässrige Säurelösung, die in dem Säuretank 52 bereits bereitgestellt wurde, über den ersten Absauganschluss 20 dem Bioreaktor 2 zugeführt. Anschließend wird auch über den zweiten Absauganschluss 24 wässrige Säurelösung dem Bioreaktor 2 zugeführt. Dies kann auch gleichzeitig mit Schritt S30 erfolgen. Alternativ ist es auch möglich, Schritt S31 vor Schritt S30 durchzuführen. Anschließend, nachdem die wässrige Säurelösung in dem Bioreaktor 2 eingeführt wurde, erfolgt in Schritt S32 eine Wartezeit. Diese beträgt vorzugsweise wenigstens 5 Minuten, vorzugsweise liegt sie in einem Bereich von 5 Minuten bis 1 Stunde, vorzugsweise 20 Minuten bis 30 Minuten. Dies ist eine ausreichende Zeit, um Kalkablagerungen zu einem Großteil zu reinigen. Anschließend oder gleichzeitig kann in Schritt S33 auch Druckluft mittels des Kompressors 92 in den Bioreaktor 2 eingeführt werden. Auch der Kompressor 92 wird von der elektronischen Steuereinheit 44 gesteuert, sodass diese in Schritt S33 ein entsprechendes Signal an diesen bereitstellt. In Schritt S34 kann dann eine Zirkulation von wässriger Säurelösung durch den Bioreaktor 2 durchgeführt werden. Hierzu wird vorzugsweise über den zweiten Absauganschluss 24 wässrige Säurelösung in den Bioreaktor 2 eingeführt und über den ersten Absauganschluss 20 abgesaugt. Hierzu öffnet die elektronische Steuereinheit 44 vorzugsweise das erste Ventil BV9, das zweite Ventil BV10, das vierte Ventil BV5 und das dritte Ventil BV3. Die Pumpe 46 wird so angetrieben, dass sie die Flüssigkeit vom ersten Pumpanschluss 64 zum zweiten Pumpanschluss 66 pumpt. Auf diese Weise kann die Zirkulation der wässrigen Säurelösung durch den Bioreaktor 2 bewirkt werden. Bei dieser Zirkulation kann zusätzlich Luft in die Flüssigkeit eingeperlt werden, vorzugsweise mittels des Kompressors 92. Die Luftblasen in der Flüssigkeit bewirken eine mechanische Reinigung auch der Leitungen.

Es ist aber auch möglich, die wässrige Säurelösung, die aus dem Bioreaktor 2 über den ersten Absauganschluss 20 abgesaugt wurde, durch die Säuredosiereinheit 62 zu leiten, um beispielsweise zusätzliche Säure einzudosieren.

In Schritt S35 wird eine Pause eingelegt und eine gewisse Zeit abgewartet. Diese Zeit dient wieder dazu, die wässrige Säurelösung einwirken zu lassen, um die Kalkablagerungen zu lösen. Sie kann einem ähnlichen Zeitbereich liegen wie oben genannt, vorzugsweise wiederum in einem Bereich von 20 bis 30 Minuten. Anschließend wird in Schritt S36 vorzugsweise wieder Druckluft über den ersten Absauganschluss 22 eingeleitet und in Schritt S37 die wässrige Säurelösung in dem Bioreaktor 2 zirkuliert. Die Schritte S35 bis S37 können sich daran dann noch mehrfach anschließen, sodass mehrere Zyklen aus Pause (Schritt S35), Einleiten von Druckluft (S36) und Zirkulieren der wässrigen Säurelösung im Bioreaktor 2 (S37) ausgeführt werden. Beispielsweise können fünf Zyklen hiervon durchgeführt werden.

In Schritt S38 dann wird die wässrige Säurelösung aus dem Bioreaktor 2 über den ersten Absauganschluss 20 abgesaugt und dem Säuretank 52 zugeführt. Dies wird mittels der Pumpe 46 bewirkt, indem das erste Ventil BV9, das fünfte Ventil BV7, das vierte Ventil BV5 geöffnet werden sowie das erste Säuretankventil BV85. Die Pumpe 46 pumpt dann die wässrige Säurelösung von dem zweiten Pumpanschluss 66 zum ersten Pumpanschluss 64 und in den Säuretank 52 hinein. Um den Bioreaktor dann von Restsäure zu reinigen, wird vorzugsweise Frischwasser sowohl in Schritt S39 über den zweiten Absauganschluss 24 zugeführt als auch in Schritt S40 über den ersten Absauganschluss 20. Optional wird auch Frischwasser über den Spülanschluss 28 eingeführt. Dieses so zum Spülen zugeführte Wasser wird vorzugsweise im Anschluss in Schritt S41 und Schritt S42 über den ersten und zweiten Absauganschluss 20, 24 abgesaugt und in den Sammeltank 50 gepumpt.

Alternativ zu dieser Neutralisierung im Säuretank 52 kann auch eine Neutralisierung im Bioreaktor 2 selbst durchgeführt werden. Hierdurch kann Frischwasser zum Spülen gespart werden.

Zu diesem Zweck wird die zu neutralisierende wässrige Säurelösung vorzugsweise zunächst aus dem Bioreaktor 2 in den Säuretank 52 abgesaugt, vorzugsweise über den ersten Absauganschluss 22. Anschließend wird vorzugsweise Frischwasser in den Bioreaktor 2 eingeführt, um diesen ein erstes Mal zu spülen. Dies kann sowohl über den Spülanschluss 28 als auch über den zweiten Absauganschluss 24 erfolgen. Die dann in dem Bioreaktor vorhandene Flüssigkeit ist sauer und muss weiter neutralisiert werden. Die Flüssigkeit kann nun über den ersten Absauganschluss 20 abgesaugt werden, und über die Säuredosiereinheit geleitet und dort mit Base versetzt werden, und zurück in den Bioreaktor 2 geführt werden. Dieser Kreislauf beziehungsweise dieses Zyklieren kann wiederholt werden, bis ein ausreichend neutraler pH-Wert erreicht wurde. Anschließend kann die neutralisierte Flüssigkeit aus dem Bioreaktor 2 abgesaugt werden, vorzugsweise über den ersten Absauganschluss 22 und dann entweder in den Sammeltank 50 geführt oder direkt in einen Kanal zum Entsorgen geleitet werden. Auf dieser Weise wird der Bioreaktor 2 nur einmal zum Spülen mit Frischwasser geflutet, wohingegen er mehrfach gespült werden müssten, wenn die zum Spülen verwendete Flüssigkeit nach jedem Spülen im Säuretank 52 neutralisiert werden müsste. Dieses Vorgehen ist besonders effizient bei mobilen Bioreaktorreinigungsanlagen, die Basekanister mit hochdosierter Base aufweisen. Bei stationären Anlagen hingegen, wird in der Regel verdünnte Base verwendet, um die Verschlauchung bzw. Verrohrung zwischen der Bioreaktorreinigungsanlage und dem Bioreaktor einfacher ausführen zu können.

Gleichzeitig oder im Anschluss kann die wässrige Säurelösung in dem Säuretank 52 neutralisiert oder zunächst auf ihren pH-Gehalt getestet werden. Wenn die wässrige Säurelösung aus dem Bioreaktor 2 über den ersten Absauganschluss 20 abgesaugt wird, ist es auch denkbar, diese nicht direkt in den Säuretank 52 zu pumpen, sondern indem die Ventile BV9, BV10, BV3 geöffnet werden, diese der Säuredosiereinheit 60 zuzuführen und von dort aus über das zweite Säuretankventil BV83 in den Säuretank 52 hinein.

Die einzelnen hier beschriebenen Schritte können auch in anderen Reihenfolgen durchgeführt werden, in anderen Kombinationen, oder mehrfach. Dies kann basierend auf Sensordaten oder Parametern, die von der elektronischen Steuereinheit 44 erfasst werden, ausgeführt werden. Beispielsweise kann sich die Anzahl der Wiederholungen eines Zirkulierens der wässrigen Säurelösung (Schritte S35 bis S37) in Abhängigkeit von dem Bioreaktortyp durchgeführt werden, welcher von der elektronischen Steuereinheit 44 von dem Bioreaktor ausgelesen wird. Weitere Parameter, die hierauf Einfluss haben können, sind auch das Zurücklegen des letzten Reinigungsintervalls, das Betriebsalter des Bioreaktors und dergleichen.

## Patentansprüche

1. Bioreaktorreinigungsanlage (1) zum Reinigen eines Bioreaktors (2), vorzugsweise eines Bioreaktors in einem Schienenfahrzeug, mit
einem ersten Absauganschluss (20) zum Anschließen an den Bioreaktor (2);
einem zweiten Absauganschluss (24) zum Anschließen an den Bioreaktor (2), über den eine Flüssigkeit aus einem Filterkorb (8) des Bioreaktors (2) absaugbar ist;
einem Spülanschluss (28) zum Zuführen einer Flüssigkeit zu einer Reinigungsdüse (10) des Bioreaktors (2);
einem Säuretank (52) zum Aufnehmen einer wässrigen Säurelösung;
einem Sammeltank (50) zum Aufnehmen von aus dem Bioreaktor (2) abgesaugter Flüssigkeit;
einem Frischwasseranschluss (42) zum Versorgen der Bioreaktorreinigungsanlage (1) mit Frischwasser;
einer Pumpe (46), vorzugsweise Drehkolbenpumpe, mit einem ersten Pumpanschluss (64) und einem zweiten Pumpanschluss (66); und
einer Messeinheit (60) zum Messen von Flüssigkeit;
wobei mittels der Pumpe (46):
- Flüssigkeit von dem ersten Absauganschluss (20) wahlweise in die Messeinheit (60), in den Sammeltank (50) oder den Säuretank (52) pumpbar ist,
- wässrige Säurelösung aus dem Säuretank (52) zum zweiten Absauganschluss (24) und/oder zum Spülanschluss (28) pumpbar ist;
- Frischwasser von dem Frischwasseranschluss (42) wahlweise zu dem Spülanschluss (28) oder der Messeinheit pumpbar ist; und
- Flüssigkeit aus der Messeinheit (60) wahlweise in den Sammeltank (50) oder zum zweiten Absauganschluss (24) pumpbar ist.

2. Bioreaktorreinigungsanlage nach Anspruch 1, aufweisend eine elektronische Steuereinheit (44) wenigstens zum Steuern der Pumpe (46), wobei die elektronische Steuereinheit (44) einen Speicher und einen Prozessor aufweist und dazu ausgebildet ist, wenigstens einen ersten Parameter von wenigstens einem Sensor der Bioreaktorreinigungsanlage und wenigstens einen zweiten Parameter von einem Benutzer über eine Mensch-Maschine-Schnittstelle (54) zu empfangen, und wobei die elektronische Steuereinheit (44) die Pumpe (46) basierend auf den ersten und zweiten Parametern ansteuert.

3. Bioreaktorreinigungsanlage nach Anspruch 1 oder 2, aufweisend eine Säuredosiereinheit (62), die wenigsten einen Säurekanister-Anschluss zum Anschließen von einem oder mehreren Säurekanistern und wenigstens einen Basekanister-Anschluss zum Anschließen von einem oder mehreren Basekanistern aufweist, und die mit dem Säuretank (52), dem Frischwasseranschluss (42) und der Pumpe (46) verbindbar ist,
wobei mittels der Pumpe (46) vorzugsweise Flüssigkeit vom Säuretank (52) zur Säuredosiereinheit pumpbar ist, und/oder wobei mittels der Pumpe (46) Flüssigkeit vom ersten Absauganschluss (20) zur Säuredosiereinheit pumpbar ist.

4. Bioreaktorreinigungsanlage nach einem der vorstehenden Ansprüche, wobei mittels der Pumpe (46) Flüssigkeit von dem Säuretank (52) zum Sammeltank (50) pumpbar ist.

5. Bioreaktorreinigungsanlage nach einem der vorstehenden Ansprüche, aufweisend ein erstes Ventil (BV9), das den ersten Absauganschluss (20) über eine erste Leitung (L1) mit einer zweiten Leitung (L2) verbindet, und vorzugsweise aufweisend ein zweites Ventil (BV10), das die zweite Leitung (L2) mit dem ersten Pumpenanschluss (64) verbindet.

6. Bioreaktorreinigungsanlage nach einem der vorstehenden Ansprüche, aufweisend ein drittes Ventil (BV8), das den zweiten Absauganschluss (24) über eine dritte Leitung (L3) mit einer vierten Leitung (L4) verbindet, und vorzugsweise aufweisend ein viertes Ventil (BV5), das die vierte Leitung (L4) mit dem zweiten Pumpenanschluss (66) verbindet.

7. Bioreaktorreinigungsanlage nach einem der vorstehenden Ansprüche, wobei der erste Pumpanschluss (64) über ein erstes Sammeltankventil (BV82) mit dem Sammeltank (50) verbunden ist, und/oder wobei der erste Pumpanschluss (64) über ein erstes Säuretankventil (BV85) mit dem Säuretank (52) verbunden ist.

8. Bioreaktorreinigungsanlage nach einem der vorstehenden Ansprüche, wobei der zweite Pumpanschluss (66) über ein zweites Sammeltankventil (BV80) mit dem Sammeltank (50) verbindbar oder verbunden ist, und/oder wobei der zweite Pumpanschluss (66) über ein zweites Säuretankventil (BV83) mit dem Säuretank (52) verbindbar oder verbunden ist.

9. Bioreaktorreinigungsanlage nach einem der vorstehenden Ansprüche, aufweisend ein erstes Messventil (BV41), das den zweiten Pumpanschluss (66) mit der Messeinheit (60) verbindet, vorzugsweise aufweisend ein zweites Messventil (BV40), das die Messeinheit (60) mit einer ersten Messleitung (ML1) verbindet, und vorzugsweise aufweisend ein erstes Frischwasserventil (BV1), das den Frischwasseranschluss (42) mit der ersten Messleitung (ML1) verbindet.

10. Bioreaktorreinigungsanlage nach Anspruch 2, aufweisend einen Füllstandsensor (LT40) in der Messeinheit (60) zum Erfassen einer in der Messeinheit (60) aufgenommenen Flüssigkeitsmenge, wobei der Füllstandsensor (LT40) mit der elektronischen Steuereinheit (44) verbunden ist und an dieser ein Messfüllstand-Signal bereitstellt.

11. Reinigungsverfahren mit einem oder mehreren Reinigungsschritten zum Reinigen und Warten eines Bioreaktors (2) mittels einer Bioreaktorreinigungsanlage (1) nach einem der Ansprüche 1 bis 10, wobei das Reinigungsverfahren umfasst:
- Absaugen von Flüssigkeit aus dem Bioreaktor (2) über einen zweiten Absauganschluss (24); und
- Pumpen der abgesaugten Flüssigkeit in einen Sammeltank (50).

12. Reinigungsverfahren nach Anspruch 11, umfassend:
- Einlesen von Daten von dem Bioreaktor (2) über eine Datenverbindung zwischen dem Bioreaktor (2) und der Bioreaktorreinigungsanlage (1); und/oder
- Einfüllen von Frischwasser über den zweiten Absauganschluss (24) in den Bioreaktor (2); und Warten einer vorbestimmten ersten Zeit; und/oder
- Zuführen von Flüssigkeit zum Spülanschluss (28) zum Zuführen der Flüssigkeit zu einer Reinigungsdüse (10) des Bioreaktors (2); und/oder
- Absaugen von Flüssigkeit aus dem Bioreaktor (2) über einen ersten Absauganschluss (20); und
- Pumpen der abgesaugten Flüssigkeit in den Sammeltank (50) oder in einen Säuretank (52).

13. Reinigungsverfahren nach einem der Ansprüche 11 bis 12, umfassend:
- Einfüllen einer wässrigen Säurelösung über den ersten Absauganschluss (20) und/oder den zweiten Absauganschluss (24) in den Bioreaktor; und
- Warten einer vorbestimmten zweiten Zeit; und
- Einleiten von Druckluft in den Bioreaktor (2) über den ersten Absauganschluss (20); und/oder
- Einfüllen einer wässrigen Säurelösung über den zweiten Absauganschluss (24) in den Bioreaktor (2); und gleichzeitig:
- Absaugen von Flüssigkeit aus dem Bioreaktor (2) über den ersten Absauganschluss (20).

14. Reinigungsverfahren nach einem der Ansprüche 11 bis 13, umfassend:
- Abmessen eines vorbestimmten Flüssigkeitsvolumens in einer Messeinheit (60);
- Zuführen des vorbestimmten Flüssigkeitsvolumens in den Bioreaktor (2) über den zweiten Absauganschluss (24);
- Warten einer vorbestimmten dritten Zeit;
- Absaugen von Flüssigkeit aus dem Bioreaktor (2) über den ersten Absauganschluss (20);
- Zuführen der abgesaugten Flüssigkeit zu der Messeinheit (60); und
- Messen des Volumens der abgesaugten Flüssigkeit.

15. Computerprogrammprodukt aufweisend Codemittel, die wenn auf einer elektronischen Steuereinheit (44) einer Bioreaktorreinigungsanlage (1) ausgeführt diese veranlassen ein Reinigungsverfahren mit einem oder mehreren Reinigungsschritten zum Reinigen und Warten des Bioreaktors (2) nach einem der Ansprüche 11 bis 14 durchzuführen.

## Claims

1. A bioreactor cleaning system (1) for cleaning a bioreactor (2), preferably a bioreactor in a rail vehicle, with
a first suction connection (20) for connecting to the bioreactor (2);
a second suction connection (24) for connecting to the bioreactor (2), via which a liquid can be suctioned out of a filter basket (8) of the bioreactor (2);
a flushing connection (28) for supplying a liquid to a cleaning nozzle (10) of the bioreactor (2);
an acid tank (52) for receiving an aqueous acid solution;
a collection tank (50) for collecting liquid extracted from the bioreactor (2);
a fresh water connection (42) for supplying the bioreactor cleaning system (1) with fresh water;
a pump (46), preferably a rotary lobe pump, having a first pump port (64) and a second pump port (66); and
a measuring unit (60) for measuring liquid;
whereby by means of the pump (46):
liquid can be pumped from the first suction connection (20) selectively into the measuring unit (60), into the collection tank (50) or into the acid tank (52),
aqueous acid solution can be pumped from the acid tank (52) to the second suction connection (24) and/or to the flushing connection (28);
fresh water can be pumped from the fresh water connection (42) selectively to the flushing connection (28) or the measuring unit; and
liquid can be pumped from the measuring unit (60) selectively into the collection tank (50) or to the second suction connection (24).

2. The bioreactor cleaning system according to claim 1, comprising an electronic control unit (44) at least for controlling the pump (46), wherein the electronic control unit (44) comprises a memory and a processor and is adapted to receive at least a first parameter from at least one sensor of the bioreactor cleaning system and at least a second parameter from a user via a human-machine interface (54), and wherein the electronic control unit (44) controls the pump (46) based on the first and second parameters.

3. The bioreactor cleaning system according to claim 1 or 2, comprising an acid dosing unit (62) having at least one acid canister connection for connecting one or more acid canisters and at least one base canister connection for connecting one or more base canisters, and being connectable to the acid tank (52), the fresh water connection (42) and the pump (46), wherein liquid can preferably be pumped from the acid tank (52) to the acid dosing unit by means of the pump (46), and/or wherein liquid can be pumped from the first suction connection (20) to the acid dosing unit by means of the pump (46).

4. The bioreactor cleaning system according to any one of the preceding claims, wherein liquid can be pumped from the acid tank (52) to the collection tank (50) by means of the pump (46).

5. The bioreactor cleaning system according to any one of the preceding claims, comprising a first valve (BV9) connecting the first suction connection (20) to a second line (L2) via a first line (L1), and preferably comprising a second valve (BV10) connecting the second line (L2) to the first pump port (64).

6. The bioreactor cleaning system according to any one of the preceding claims, comprising a third valve (BV8) connecting the second suction connection (24) to a fourth line (L4) via a third line (L3), and preferably comprising a fourth valve (BV5) connecting the fourth line (L4) to the second pump port (66).

7. The bioreactor cleaning system according to any one of the preceding claims, wherein the first pump port (64) is connected to the collection tank (50) via a first collection tank valve (BV82), and/or wherein the first pump port (64) is connected to the acid tank (52) via a first acid tank valve (BV85).

8. The bioreactor cleaning system according to any one of the preceding claims, wherein the second pump port (66) is connectable or connected to the collection tank (50) via a second collection tank valve (BV80), and/or wherein the second pump port (66) is connectable or connected to the acid tank (52) via a second acid tank valve (BV83).

9. The bioreactor cleaning system according to any one of the preceding claims, comprising a first measuring valve (BV41) connecting the second pump port (66) to the measuring unit (60), preferably comprising a second measuring valve (BV40) connecting the measuring unit (60) to a first measuring line (ML1), and preferably comprising a first fresh water valve (BV1) connecting the fresh water connection (42) to the first measuring line (ML1).

10. The bioreactor cleaning system according to claim 2, comprising a level sensor (LT40) in the measuring unit (60) for detecting a quantity of liquid received in the measuring unit (60), wherein the level sensor (LT40) is connected to and provides a measuring level signal to the electronic control unit (44).

11. A cleaning method comprising one or more cleaning steps for cleaning and maintaining a bioreactor (2) by means of a bioreactor cleaning system (1) according to any one of claims 1 to 10, wherein the cleaning method comprises:
suctioning liquid from the bioreactor (2) via a second suction connection (24); and
pumping the extracted liquid into a collection tank (50).

12. The cleaning method according to claim 11, comprising:
reading data from the bioreactor (2) via a data link between the bioreactor (2) and the bioreactor cleaning system (1); and/or
filling fresh water into the bioreactor (2) via the second suction connection (24); and waiting a predetermined first time; and/or
supplying liquid to the flushing connection (28) for supplying the liquid to a cleaning nozzle (10) of the bioreactor (2); and/or
suctioning liquid from the bioreactor (2) via a first suction connection (20); and
pumping the extracted liquid into the collection tank (50) or into an acid tank (52).

13. The cleaning method according to any one of claims 11 to 12, comprising:
filling an aqueous acid solution into the bioreactor via the first suction connection (20) and/or the second suction connection (24); and
waiting a predetermined second time; and
introducing compressed air into the bioreactor (2) via the first suction connection (20); and/or
filling an aqueous acid solution into the bioreactor (2) via the second suction connection (24); and simultaneously:
suctioning liquid from the bioreactor (2) via the first suction connection (20).

14. The cleaning method according to any one of claims 11 to 13, comprising:
measuring a predetermined volume of liquid in a measuring unit (60);
supplying the predetermined volume of liquid into the bioreactor (2) via the second suction connection (24);
waiting a predetermined third time;
suctioning liquid from the bioreactor (2) via the first suction connection (20);
supplying the extracted liquid to the measuring unit (60); and
measuring the volume of the extracted liquid.

15. A computer program product comprising code means which, when executed on an electronic control unit (44) of a bioreactor cleaning system (1), cause it to perform a cleaning method comprising one or more cleaning steps for cleaning and maintaining the bioreactor (2) according to any one of claims 11 to 14.

## Revendications

1. Système de nettoyage de bioréacteur (1) pour nettoyer un bioréacteur (2), de préfé-rence un bioréacteur dans un véhicule ferroviaire, avec un premier raccord d'aspiration (20) pour se raccorder au bioréacteur (2) ;
un deuxième raccord d'aspiration (24) pour se raccorder au bioréacteur (2), par lequel un liquide peut être aspiré d'un panier filtrant (8) du bioréacteur (2) ;
un raccord de rinçage (28) pour alimenter une buse de nettoyage (10) du bioréacteur (2) en liquide ;
un réservoir d'acide (52) pour recevoir une solution acide aqueuse ;
un réservoir collecteur (50) pour recueillir le liquide extrait du bioréacteur (2) ;
un raccord d'eau fraîche (42) pour alimenter le système de nettoyage de bioréacteur (1) en eau fraîche ;
une pompe (46), de préférence une pompe à lobes rotatifs, ayant un premier orifice de pompe (64) et un deuxième orifice de pompe (66) ; et
une unité de mesure (60) pour mesurer le liquide ;
dans lequel au moyen de la pompe (46) :
du liquide peut être pompé du premier raccord d'aspiration (20) sélectivement dans l'unité de mesure (60), dans le réservoir collecteur (50) ou dans le réservoir d'acide (52),
de la solution acide aqueuse peut être pompée du réservoir d'acide (52) vers le deuxième raccord d'aspiration (24) et/ou vers le raccord de rinçage (28) ;
de l'eau fraîche peut être pompée du raccord d'eau fraîche (42) sélectivement vers le raccord de rinçage (28) ou vers l'unité de mesure ; et
du liquide peut être pompé de l'unité de mesure (60) sélectivement dans le réser-voir collecteur (50) ou vers le deuxième raccord d'aspiration (24).

2. Système de nettoyage de bioréacteur selon la revendication 1, comprenant une unité de commande électronique (44) destinée au moins à commander la pompe (46), dans lequel l'unité de commande électronique (44) comprend une mémoire et un processeur et est conçue pour recevoir au moins un premier paramètre d'au moins un capteur du système de nettoyage de bioréacteur et au moins un deuxième paramètre d'un utilisateur via une interface homme-machine (54), et dans lequel l'unité de commande électronique (44) commande la pompe (46) sur la base des premier et deuxième paramètres.

3. Système de nettoyage de bioréacteur selon la revendication 1 ou 2, comprenant une unité de dosage d'acide (62) qui comprend au moins un raccord de bidon d'acide pour raccorder un ou plusieurs bidons d'acide et au moins un raccord de bidon de base pour raccorder un ou plusieurs bidons de base, et qui est raccordable au réservoir d'acide (52), au raccord d'eau fraîche (42) et à la pompe (46), du liquide pouvant de préférence être pompé du réservoir d'acide (52) vers l'unité de dosage d'acide au moyen de la pompe (46), et/ou du liquide pouvant être pompé du premier raccord d'aspiration (20) vers l'unité de dosage d'acide au moyen de la pompe (46).

4. Système de nettoyage de bioréacteur selon l'une des revendications précédentes, dans lequel du liquide peut être pompé du réservoir d'acide (52) vers le réservoir collecteur (50) au moyen de la pompe (46).

5. Système de nettoyage de bioréacteur selon l'une des revendications précédentes, comprenant une première vanne (BV9) reliant le premier raccord d'aspiration (20) à une deuxième conduite (L2) par l'intermédiaire d'une première conduite (L1), et comprenant de préférence une deuxième vanne (BV10) reliant la deuxième conduite (L2) au premier orifice de pompe (64).

6. Système de nettoyage de bioréacteur selon l'une des revendications précédentes, comprenant une troisième vanne (BV8) reliant le deuxième raccord d'aspiration (24) à une quatrième conduite (L4) par l'intermédiaire d'une troisième conduite (L3), et comprenant de préférence une quatrième vanne (BV5) reliant la quatrième conduite (L4) au deuxième orifice de pompe (66).

7. Système de nettoyage de bioréacteur selon l'une des revendications précédentes, dans lequel le premier orifice de pompe (64) est relié au réservoir collecteur (50) par l'in-termédiaire d'une première vanne de réservoir collecteur (BV82), et/ou dans lequel le premier orifice de pompe (64) est relié au réservoir d'acide (52) par l'intermédiaire d'une première vanne de réservoir d'acide (BV85).

8. Système de nettoyage de bioréacteur selon l'une des revendications précédentes, dans lequel le deuxième orifice de pompe (66) est raccordable ou raccordé au réservoir collecteur (50) par l'intermédiaire d'une deuxième vanne de réservoir collecteur (BV80), et/ou dans lequel le deuxième orifice de pompe (66) est raccordable ou raccordé au réservoir d'acide (52) par l'intermédiaire d'une deuxième vanne de réservoir d'acide (BV83).

9. Système de nettoyage de bioréacteur selon l'une des revendications précédentes, comprenant une première vanne de mesure (BV41) reliant le deuxième orifice de pompe (66) à l'unité de mesure (60), comprenant de préférence une deuxième vanne de mesure (BV40) reliant l'unité de mesure (60) à une première conduite de mesure (ML1), et comprenant de préférence une première vanne d'eau fraîche (BV1) reliant le raccord d'eau fraîche (42) à la première conduite de mesure (ML1).

10. Système de nettoyage de bioréacteur selon la revendication 2, comprenant un capteur de niveau (LT40) dans l'unité de mesure (60) pour détecter une quantité de liquide reçue dans l'unité de mesure (60), dans lequel le capteur de niveau (LT40) est relié à l'unité de commande électronique (44) et lui fournit un signal de niveau de mesure.

11. Procédé de nettoyage comprenant une ou plusieurs étapes de nettoyage pour nettoyer et entretenir un bioréacteur (2) au moyen d'un système de nettoyage de bioréacteur (1) selon l'une des revendications 1 à 10, dans lequel le procédé de nettoyage comprend :
l'aspiration de liquide du bioréacteur (2) via un deuxième raccord d'aspiration (24) ; et
le pompage du liquide aspiré dans un réservoir collecteur (50).

12. Procédé de nettoyage selon la revendication 11, comprenant :
la lecture de données du bioréacteur (2) via une liaison de données entre le bio-réacteur (2) et le système de nettoyage de bioréacteur (1) ; et/ou
l'introduction d'eau fraîche dans le bioréacteur (2) via le deuxième raccord d'aspiration (24) ; et l'attente d'un premier délai prédéterminé ; et/ou
l'alimentation en liquide du raccord de rinçage (28) pour alimenter la buse de nettoyage (10) du bioréacteur (2) en liquide ; et/ou
l'aspiration de liquide du bioréacteur (2) via un premier raccord d'aspiration (20) ; et
le pompage du liquide aspiré dans le réservoir collecteur (50) ou dans un réser-voir d'acide (52).

13. Procédé de nettoyage selon l'une des revendications 11 à 12, comprenant :
l'introduction d'une solution acide aqueuse dans le bioréacteur via le premier raccord d'aspiration (20) et/ou le deuxième raccord d'aspiration (24) ; et
l'attente d'un deuxième délai prédéterminé ; et
l'introduction d'air comprimé dans le bioréacteur (2) via le premier raccord d'aspi-ration (20) ; et/ou
l'introduction d'une solution acide aqueuse dans le bioréacteur (2) via le deuxième raccord d'aspiration (24) ; et simultanément :
l'aspiration de liquide du bioréacteur (2) via le premier raccord d'aspiration (20).

14. Procédé de nettoyage selon l'une des revendications 11 à 13, comprenant :
la mesure d'un volume de liquide prédéterminé dans une unité de mesure (60) ;
l'alimentation du bioréacteur (2) en ledit volume de liquide prédéterminé via le deuxième raccord d'aspiration (24) ;
l'attente d'un troisième délai prédéterminé ;
l'aspiration de liquide du bioréacteur (2) via le premier raccord d'aspiration (20) ;
l'alimentation de l'unité de mesure (60) en liquide aspiré ; et
la mesure du volume du liquide aspiré.

15. Produit-programme d'ordinateur comprenant des moyens de code qui, lorsqu'ils sont exécutés sur une unité de commande électronique (44) d'un système de nettoyage de bioréacteur (1), amènent celui-ci à exécuter un procédé de nettoyage comprenant une ou plusieurs étapes de nettoyage pour nettoyer et entretenir le bioréacteur (2) selon l'une des revendications 11 à 14.
